# EUROPEAN PATENT SPECIFICATION

(11) **EP 4 404 956 B1**
(45) Date of publication and mention of the grant of the patent: **01.04.2026**
(21) Application number: 22783553.5
(22) Date of filing: 20.09.2022
(51) Int. Cl.: A61K 39/00, A61K 35/17, A61K 38/00, A61P 35/02, C07K 14/725, C07K 16/28, C07K 16/40

(54) **BINDING PROTEINS FOR TERMINAL DEOXYNUCLEOTIDYL TRANSFERASE (TDT)**
BINDUNGSPROTEINE FÜR ENDDEOXYNUCLEOTIDYLTRANSFERASE (TDT)
PROTÉINES DE LIAISON POUR DÉSOXYNUCLÉOTIDYL TRANSFÉRASE TERMINALE (TDT)

(30) Priority: 21.09.2021 GB 202113437
(43) Date of publication of application: 31.07.2024
(73) Proprietor: Oslo Universitetssykehus HF, 0424 Oslo (NO); Universitetet I Oslo, 0316 Oslo (NO)
(72) Inventor: OLWEUS, Johanna, 0316 Oslo (NO); ALI, Muhammad, 0316 Oslo (NO)
(74) Representative: Dehns
(86) International application number: PCT/GB2022/052368
(87) International publication number: WO 2023/047089

(56) References cited:
- WO-A1-2017/174645
- US-A1- 2011 142 842
- ALI MUHAMMAD ET AL: "T cells targeted to TdT kill leukemic lymphoblasts while sparing normal lymphocytes", NATURE BIOTECHNOLOGY, NATURE PUBLISHING GROUP US, NEW YORK, vol. 40, no. 4, 6 December 2021 (2021-12-06), pages 488 - 498, XP037799132, ISSN: 1087-0156, [retrieved on 20211206], DOI: 10.1038/S41587-021-01089-X

## Description

### Field

The present disclosure concerns binding proteins based on sequences from T cell receptors (TCRs). The binding proteins recognise fragments of human terminal deoxynucleotidyl transferase (TdT) in the context of a major histocompatibility complex (MHC) class I comprising the human leukocyte antigen A2 (HLA-A2 or HLA-A*02). The binding proteins disclosed herein, and cells expressing them, may be used in cancer therapy.

### Background

Cancer treatment based on autologous lymphocytes expressing chimeric antigen receptors (CARs) is well known. A variety of CARs targeting tumour-associated antigens have been disclosed in the last decade. However, a major drawback of conventional CARs is their ability to target cell surface proteins only.

Acute lymphoblastic leukaemia (ALL) is a blood cancer for which new therapies are urgently needed. In B cell ALL (B-ALL), targeting of the B cell-specific antigen CD19 with CAR T cell therapy frequently induces complete remission. However, approximately 40-50 % of B-ALL patients relapse following anti-CD19 CAR-T cell therapy (Maude et al., New England Journal of Medicine 378: 439-448, 2018; S. A. Grupp et al., Blood 132, 895-895 (2018)), most frequently due to loss of CD19 from the cancer (Shah & Fry, Nature Reviews Clinical Oncology 16: 372-385, 2019). Moreover, since CD19 is not a tumour-specific antigen, normal and malignant B cells alike are killed by anti-CD19 CAR-T cell therapy. Although B cell aplasia is relatively well tolerated, it necessitates life-long immunoglobulin substitution, and the long-term effects of persistent CD19-specific CAR-T cells are unknown. Treatments that preserve normal B-lymphopoiesis, without the toxicities associated with allogeneic haematopoietic stem cell transplantation, would thus be desirable.

In spite of a multitude of T-cell specific markers, no CAR therapy is approved for T cell ALL (T-ALL) that efficiently targets malignant cells across all T-ALL sub-types, and at the same time spares mature, healthy T cells. This would be crucial to accomplish, as depletion of healthy T cells is prohibitively toxic or even incompatible with life. Moreover, no tumour-specific target has been identified in T-ALL, and no immunotherapy has yet proven effective in clinical trials. In the event of failed chemotherapy (15-20 %), T-ALL has a dismal prognosis with overall survival less than 25 % (Sellar et al., British Journal of Haematology 181: 515-522, 2018).

The present inventors have identified the intracellular enzyme terminal deoxynucleotidyl transferase (TdT) as a target for immunotherapy in B-ALL and T-ALL. The function of TdT is to add N-nucleotides to V-D-J junctions during recombination of the T cell receptor (TCR) and B cell receptor genes (Komori et al., Science 261: 1171-1175, 1993), and accordingly expression of TdT is confined to the B and T cell lineages. TdT is overexpressed in 80-94 % of ALL and lymphoblastic lymphoma of B and T cell origin (Drexler et al., Acta Haematologia 75: 12-17, 1986) but is not expressed in haematopoietic stem cells (Pellin et al., Nature Communications 10: 2395, 2019), and so myelopoiesis, including erythro- and thrombopoiesis, should be unaffected by targeting of TdT, and since TdT is downregulated during differentiation (Li et al., Journal of Experimental Medicine 178: 951-960, 1993) normal mature B and T cells should be spared. The present inventors have generated TCRs that recognise TdT-derived peptides which can be used in ALL therapy. TdT has previously been identified as a potential target for cancer immunotherapy (US 2017/0290897) but no current therapeutics targeting TdT are known.

The TCRs provided herein target cancerous T and B cells in ALL, but only a small subset of healthy T and B cells. The TCRs provided herein thus provide a first immunotherapy for treatment of a majority of T-ALL sub-types, and an improved immunotherapy for B-ALL that does not cause lifelong immune impairment.

US2011/142842 teaches T-cell receptors which recognize TdT-peptide ALYDKTKRI presented in an HLA-A*0201 context. WO2017/174645 teaches T-cell receptors which recognize TdT-peptide ALYDKTKRIFL presented by a MHC molecule.

### Summary

The present disclosure provides binding proteins capable of specific binding to a human leukocyte antigen complex class I presenting a TdT-peptide as set forth in SEQ ID NO: 1 or SEQ ID NO: 15. Such binding proteins (or cells expressing them) may be used for targeting cancer cells expressing TdT, in particular ALL. As demonstrated in the examples, the claimed binding proteins have excellent specificity for HLA-A*02:01 presenting either the TdT-peptide set forth in SEQ ID NO: 1 or the TdT-peptide set forth in SEQ ID NO: 15 under physiological conditions. Notably, HLA-A*02:01 is expressed by a major fraction of patients of European, Middle Eastern or North African ancestry, suffering from ALL. Accordingly, there is provided a novel therapy for T-ALL and B-ALL, which may be of particular benefit for chemotherapy-resistant patients.

Thus, in a first aspect, provided herein is a binding protein capable of specific binding to a human leukocyte antigen (HLA) complex type A2 presenting a peptide having the amino acid sequence ALYDKTKRIFL set forth in SEQ ID NO: 15;
wherein the protein comprises an antigen binding unit comprising an α-chain variable domain and a β-chain variable domain;
wherein the α-chain variable domain comprises three complementarity determining regions (CDRs): CDR1, CDR2 and CDR3 which respectively comprise the amino acid sequences set forth in SEQ ID NOs: 16, 17 and 18; and
the β-chain variable domain comprises three CDRs: CDR1, CDR2 and CDR3 which respectively comprise the amino acid sequences set forth in SEQ ID NOs: 19, 20 and 21.

In a second aspect, provided herein is a binding protein capable of specific binding to a human leukocyte antigen (HLA) complex type A2 presenting a peptide having the amino acid sequence ALYDKTKRI set forth in SEQ ID NO: 1;
wherein the protein comprises an antigen binding unit comprising an α-chain variable domain and a β-chain variable domain;
wherein the α-chain variable domain comprises three complementarity determining regions (CDRs): CDR1, CDR2 and CDR3 which respectively comprise the amino acid sequences set forth in SEQ ID NOs: 2, 3 and 4; and
the β-chain variable domain comprises three CDRs: CDR1, CDR2 and CDR3 which respectively comprise the amino acid sequences set forth in SEQ ID NOs: 5, 6 and 7.

In a third aspect there is provided a recombinant nucleic acid molecule encoding a binding protein as provided herein.

In a fourth aspect there is provided a vector comprising a recombinant nucleic acid molecule as provided herein.

A fifth aspect provides a kit comprising a first recombinant nucleic acid molecule encoding a first chain of a binding protein and a second recombinant nucleic acid molecule encoding a second chain of a binding protein, wherein:
(i) the first chain comprises an α-chain variable domain of the first aspect of the disclosure and the second chain comprises a β-chain variable domain of the first aspect of the disclosure; or
(ii) the first chain comprises an α-chain variable domain of the second aspect of the disclosure and the second chain comprises a β-chain variable domain of the second aspect of the disclosure.

A sixth aspect provides an immune effector cell comprising a recombinant nucleic acid molecule as provided herein, a vector as provided herein, or a pair of recombinant nucleic acid molecules as comprised in a kit as provided herein, and expressing a binding protein as provide herein in its cell membrane.

A seventh aspect provides a pharmaceutical composition comprising an immune effector cell as provided herein.

An eighth aspect provides an immune effector cell as provided herein or a pharmaceutical composition as provided herein for use in therapy.

A ninth aspect provides an immune effector cell as provided herein or a pharmaceutical composition as provided herein for use in the treatment of cancer, wherein the cancer expresses terminal deoxynucleotidyl transferase (TdT).

Similarly, there is provided a method of treating cancer in a subject, wherein the cancer expresses terminal deoxynucleotidyl transferase (TdT), the method comprising administering an immune effector cell as provided herein or a pharmaceutical composition as provided herein to the subject.

Also provided is the use of an immune effector cell as provided herein in the manufacture of a medicament for the treatment of cancer, wherein the cancer expresses terminal deoxynucleotidyl transferase (TdT).

The cancer treated according to the disclosures herein may in particular be (but not limited to) acute lymphoblastic leukaemia (ALL), for instance B cell acute lymphoblastic leukaemia (B-ALL) or T cell acute lymphoblastic leukaemia (T-ALL).

In a tenth aspect, there is provided a method of generating a terminal deoxynucleotidyl transferase (TdT)-specific immune effector cell, the method comprising introducing a recombinant nucleic acid molecule as provided herein, a vector as provided herein, or a pair of recombinant nucleic acid molecules as comprised in a kit as provided herein, into the immune effector cell.

### Brief Description of the Figures

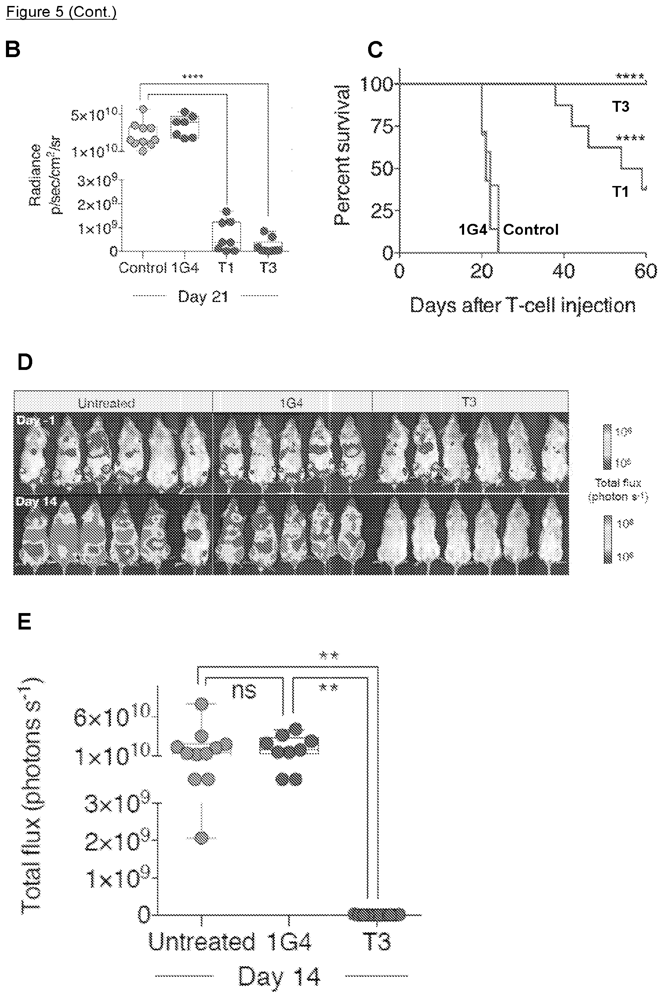

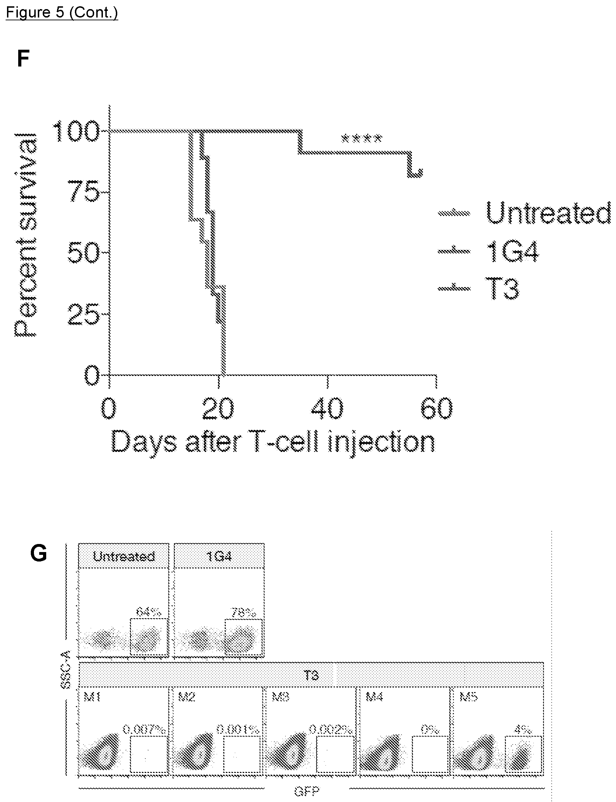

*Figure 1**: Schematic Diagrams of Various TCR Constructs*
   Figure 1A shows a full-length TCR comprising an α-chain and a β-chain. Both chains comprise a variable domain with 3 CDRs (lighter rectangles), an extracellular constant domain, a transmembrane domain and a short cytoplasmic domain.
   Figure 1B shows a minimal binding unit comprising an α-chain variable domain with 3 CDRs (lighter rectangles) and a β-chain variable domain with 3 CDRs (lighter rectangles).
   Figure 1C shows a binding unit comprising an α-chain variable domain with 3 CDRs (lighter rectangles) and a β-chain variable domain with 3 CDRs (lighter rectangles) wherein the β-chain variable domain is connected to the α-chain variable domain via a peptide linker (referred to herein as a TCR-scFv).
   Figure 1D shows a receptor comprising a binding unit, an extracellular constant domain, a transmembrane domain and a cytoplasmic signalling domain (referred to herein as a chimeric TCR).
   Figure 1E shows a truncated TCR comprising an α-chain and a β-chain, the receptor comprising a binding unit, extracellular constant domains and two cysteine bridges (referred to herein as a soluble TCR).
   Figure 1F shows a receptor comprising the structure from Figure 1E, in which one of the chains further comprises a transmembrane domain and a cytoplasmic signalling domain (referred to herein as a TCR-CAR).
*Figure 2* *- TdT-Reactive TCRs are Restricted to HLA-A2*
   Figure 2A shows activation of T1 and T3 cells following co-incubation with peptide-pulsed T2 lymphoblast cells. EC₅₀ = half maximal effective concentration. Data are pooled from 3 independent experiments, where each circle represents the mean of 3 technical replicates from an individual experiment. Error bars show SD.
   Figure 2B shows CD137 up-regulation on CD8+ T1 and T3 cells after co-culture with EBV-LCLs derived from one HLA-A2^{pos} and one HLA-A2^{neg} donor. The cell lines were pulsed with indicated concentrations of peptide-1 (T1) or peptide-3 (T3), or electroporated with mRNA encoding full-length TdT. Data points represent three technical replicates in one experiment representative of two performed.
   Figure 2C shows activation of T1 and T3 cells after co-culture with various cell lines with indicated HLA-A2 and TdT expression, loaded or not with TdT peptides (2 x 10⁻⁷ M). The suffix +A2 denotes cell lines transduced with HLA-A*02:01. Results are from one experiment representative of 2 or 3 performed with different T-cell donors, data points represent technical replicates (2-3) and error bars show range.
*Figure 3**: TdT-Reactive TCRs Show no Off- Target Reactivity*
   Figure 3A shows heat maps of IFN-γ concentration in culture supernatants of T1 (top) and T3 cells (bottom) co-incubated with EBV-LCLs pulsed with peptides from mimotope libraries (peptide concentration: 2 x 10⁻⁷ M). Column/row intersections indicate the replaced amino acid at a given position and white circles indicate the amino acid in the wild-type peptide. The IFN-γ concentration range for positive reactions was 500-31254 pg/mL. One replicate per condition.
   Figures 3B-C show IFN-γ production by the T1 (B) and T3 (C) cells after co-culture with target cells loaded with the indicated peptides. 9-mer and 11-mer peptides were included that contained amino acids upstream or downstream of the wild type peptide-1 and -3 in the TdT protein sequence. In addition, 8 to 12 amino acid long peptides were included that contained parts or all of peptide-1 and -3. One replicate per condition.
*Figure 4**: T1 and T3 cells are Activated by, and Effectively Kill, TdT^{pos} HLA-A2^{pos} Leukaemia Cell Lines*
   Figure 4A shows viable Td^{pos}HLA-A2^{pos} NALM-6 and BV173 cells after 48 h of co-culture with T1 and T3 cells (E:T ratio of 1:1), in percent of corresponding numbers following treatment with mock-transduced T cells, quantified by flow cytometry. Data points represent technical replicates in one experiment representative of 3 performed.
   Figure 4B shows flow cytometry plots of BV173 cells co-cultured with mock, T1 and T3 cells for 48 h. Inset numbers display event counts within the live tumour cell gate.
*Figure 5**: T1 and T3 Cells Efficiently Kill Leukaemia Cells in vitro and in vivo in BV173 and NALM-6 Animal Models*
   Figures 5A-B and D-E show bioluminescence imaging of leukaemia-bearing mice one day before, and 21 days (BV173, A-B) or 14 days (NALM-6, D-E) after treatment with human T cells transduced with 1G4 (reactive to NY-ESO-1), T1 or T3. Untreated mice were included as controls.
   Figures 5C and 5F show a survival analysis of leukaemia-bearing mice that were either untreated (n=10 (BV173) or n=11 (NALM-6)), or treated with 1G4 (n=7(BV173) or n=9 (NALM-6)), T1 (n=8 (BV173)) or T3 (n=9 (BV173) or n=11 (NALM-6)) cells.
   Data shown in Figs 5B and C and Figs 5E and F are pooled from two independent experiments. Box plots in Figs 5B and E show the interquartile range (25th to 75th percentile) with the central bar indicating the median and whiskers indicating the range. Dots represent data from individual mice. *P* = not significant (ns), ***P* < 0.01, *****P* < 0.0001, calculated by ordinary one-way ANOVA with adjustment for multiple comparisons with Tukey's post-test. Survival analysis (Figs 5C and F) was performed by two-sided Log-rank (Mantel-Cox) test, *****P* < 0.0001.
   Figure 5G shows flow cytometry plots showing bone-marrow tumour burden in T3-treated mice on day 60 (M1-M5), compared to the 1G4-treated or untreated mice at time of sacrifice (day 21). Threshold for positive leukaemia detection was set as GFP⁺ cells ≥ 0.01 % of live viable cells.

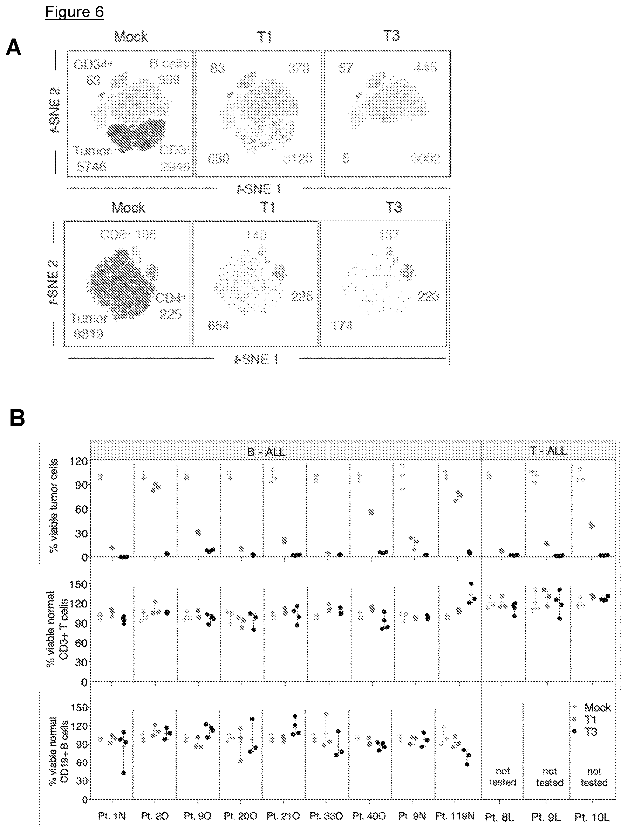

*Figure 6**: T1 and T3 Cells Deplete Primary Cancer Cells while Sparing Mature B and T Lymphocytes and Non-Lineage Committed Haematopoietic Progenitor Cells*
   Figure 6A shows representative t-distributed stochastic neighbour embedding (t-SNE) plots showing live HLA-A2^{pos},Td^{pos} B-ALL tumour cells (CD19⁺CD10⁺ events, left panel) and T-ALL tumour cells (CD5⁺CD7⁺CD99⁺ and surface CD3⁻CD4⁻ events, right panel), normal B cells (CD19⁺CD10⁻), normal T cells (CD3⁺ and CD8⁺ or CD4⁺) and CD34⁺lin⁻ progenitor cells following 72 h of co-culture with mock-, T1- or T3-transduced T cells (E:T ratio = 1:1), as quantified by flow cytometry. TCR-transduced cells were excluded from analysis as CTV positive events.
   Figure 6B shows diagnostic samples from 12 patients with HLA-A2^{pos},Td/T^{pos} B-ALL or T-ALL, assayed as described in 6A. Each dot represents the number of live tumour cells, normal B cells or T cells, after co-culture with T1 (green) or T3 (purple) cells, in percent of corresponding numbers in cultures treated with mock-transduced T cells. Data points represent 3 or 4 technical replicates and error bars show range. Data shown are from one experiment representative of at least two performed for each patient sample.

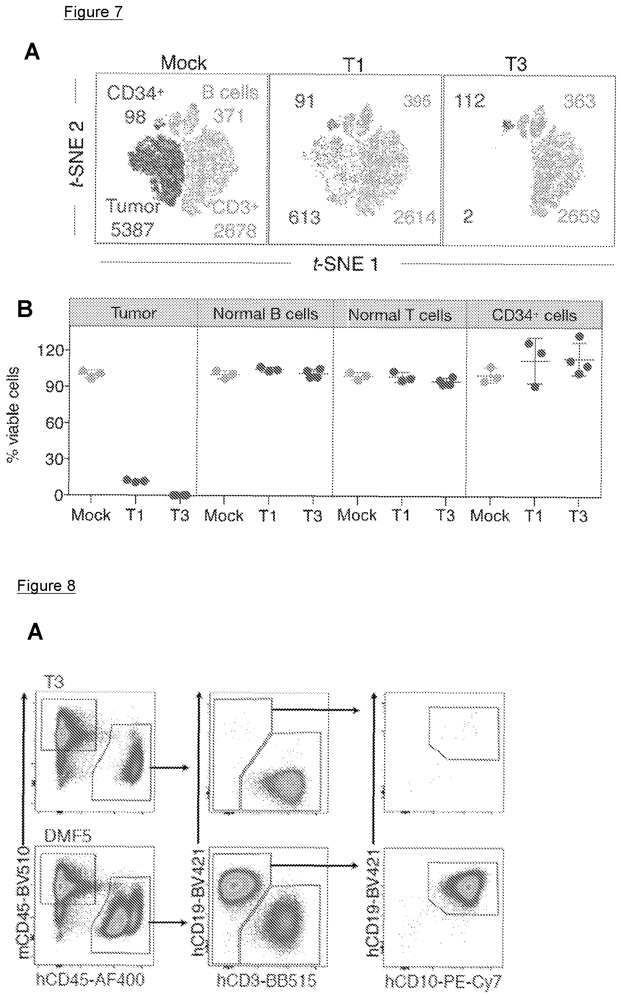

*Figure 7**: Patient-Derived CD8+ T Cells Transduced with T1 or T3 Efficiently Kill Autologous B-ALL Cells*
   Figure 7A shows a t-SNE plot of peripheral blood diagnostic sample from a B-ALL patient after 72 h of co-culture with autologous T cells transduced with T1, T3 or mock. Inset numbers denote absolute event counts of the indicated cell populations after co-culture with mock, T1 and T3 cells.
   Figure 7B shows quantification of malignant cells, normal B, T and CD34+lin-progenitor cells after performing the flow cytometry-based cytotoxicity assay for 72 h. Data points represents technical replicates (3-4) from one representative experiment out of 2 performed and error bars show range.

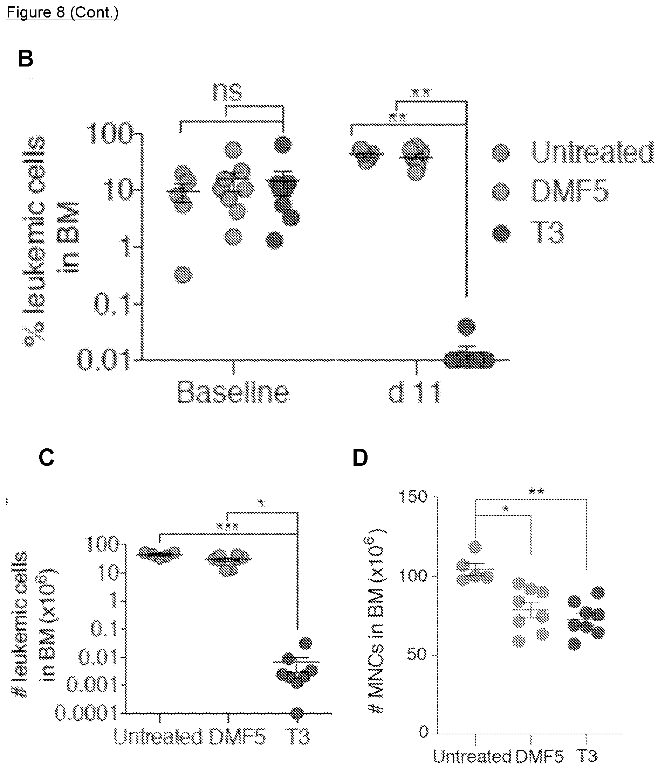

*Figure 8**: T3 Cells Efficiently Eliminate Primary B-ALL Cells While Sparing Healthy Haematopoiesis in vivo*
   In Figures 8A to 8C untreated *n* = 5, DMF5-treated *n* = 8, T3-treated *n* = 8. The figure shows pooled data from two experiments.
   Figure 8A shows representative FACS plots of viable single mononuclear cells (MNC) from bone marrow (BM) of T3-treated (top) and DMF5-treated (bottom) NSG mice engrafted with primary human B-ALL cells.
   Figure 8B shows percentage leukaemic cells (hCD45⁺CD19⁺CD10⁺) in BM adjusted for human T cells at baseline, and at day 11 after T cell infusions.
   Figure 8C shows number of leukaemic cells present in BM.
   Figure 8D show the number of MNCs in BM. Data are pooled from two independent experiments and presented as mean ± SEM at terminal analysis 11 days post treatment of untreated, DMF5-treated and T3-treated mice. Populations were identified by flow cytometry according to the gating strategy shown in Fig. 8A. Kruskal-Wallis ANOVA by Dunn's multiple comparisons test was performed using GraphPad Prism software for statistical analyses (**P <* 0.05, ***P <* 0.01, ****P <* 0.001).

### Detailed Description

The expression profile of TdT is described above. As detailed, during normal haematopoiesis, both B and T cell lineages express TdT transiently, but mature B and T cells, and CD34+ haematopoietic stem cells, lack TdT expression. This allows targeting of cancerous TdT-expressing cells while sparing haematopoietic stem cells and mature lymphocytes. Successful treatment targeting TdT would thus leave patients with healthy B cell and T cell compartments, and thus preserve their adaptive immune responses. As detailed above, TdT is overexpressed in most ALL. As TdT is localised intracellularly, it cannot be targeted by conventional CARs utilising targeting units from antibodies. However, the TCRs and TCR-derived binding proteins provided herein recognise TdT-derived peptides when presented by MHC class I molecules, and thus are able to target TdT-expressing cells.

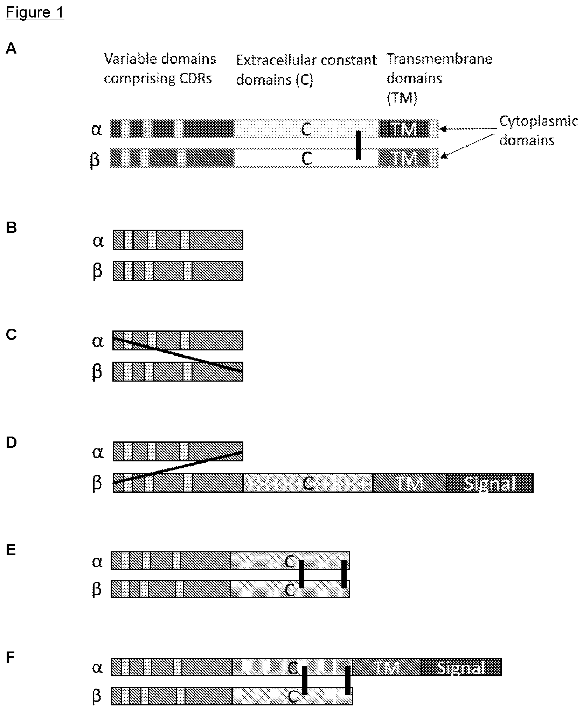

The binding proteins provided herein are derived from TCRs referred to herein as the T1 and T3 TCRs. The T1 and T3 TCRs are both αβ TCRs, i.e. heterodimers which comprise an α-chain and a β-chain. The general structure of αβTCRs is well known in the art, and schematically represented in **Figure 1A****.** The binding proteins may be provided in a variety of formats, including the classical, or "native", full-length 2-chain surface receptor format (e.g. αβ format), or a format with a single chain antigen binding unit, or as a soluble molecule etc.

As is well known, the variable domains of the α- and β-chains each comprise three complementarity-determining regions (CDRs) numbered CDR1, CDR2 and CDR3 (starting from the N-terminus). It is the CDRs which directly interact with a target epitope/MHC complex, and thus determine the specificity of a TCR, with CDR3 being the most important CDR in determining TCR specificity. The CDRs are separated and flanked by framework sequences, which form a scaffold for the CDRs, such that their spatial arrangement is appropriate for target binding.

The T3 TCR recognises the peptide having the amino acid sequence ALYDKTKRIFL, set forth in SEQ ID NO: 15, when presented by a Class I MHC comprising HLA-A2. SEQ ID NO: 15 corresponds to amino acids 475-485 of human TdT (UniProt entry P04053, SEQ ID NO: 27). The α-chain of the T3 TCR comprises a variable region in which CDR1 (which may be referred to as VαCDR1) has the amino acid sequence TSINN, set forth in SEQ ID NO: 16; VαCDR2 has the amino acid sequence IRSNERE, set forth in SEQ ID NO: 17; and VαCDR3 has the amino acid sequence CATDAGAYSGGGADGLTF, set forth in SEQ ID NO: 18. The β-chain of the T3 TCR comprises a variable region in which CDR1 (which may be referred to as VβCDR1) has the amino acid sequence MNHEY, set forth in SEQ ID NO: 19; VβCDR2 has the amino acid sequence SMNVEV, set forth in SEQ ID NO: 20; and VβCDR3 has the amino acid sequence CASSLSSSYNEQFF, set forth in SEQ ID NO: 21.

The T1 TCR recognises the peptide having the amino acid sequence ALYDKTKRI, set forth in SEQ ID NO: 1, when presented by a Class I MHC comprising HLA-A2. SEQ ID NO: 1 corresponds to amino acids 475-483 of human TdT. The α-chain of the T1 TCR comprises a variable region in which VαCDR1 has the amino acid sequence VSGLRG, set forth in SEQ ID NO: 2; VαCDR2 has the amino acid sequence LYSAGEE, set forth in SEQ ID NO: 3; and VαCDR3 has the amino acid sequence CAVQASSNSGYALNF, set forth in SEQ ID NO: 4. The β-chain of the T1 TCR comprises a variable region in which VβCDR1 has the amino acid sequence SQVTM, set forth in SEQ ID NO: 5; VβCDR2 has the amino acid sequence ANQGSEA, set forth in SEQ ID NO: 6; and VβCDR3 has the amino acid sequence CSVEPGYADTQYF, set forth in SEQ ID NO: 7.

Thus the first aspect of the present disclosure provides a binding protein as described above, based on the T3 TCR.

The α-chain and β-chain of the T3 TCR have variable domains comprising the amino acid sequences set forth in SEQ ID NO: 22 and SEQ ID NO: 23, respectively. In a particular embodiment, the binding protein based on the T3 TCR comprises an antigen binding unit comprising an α-chain variable domain comprising or consisting of the amino acid sequence set forth in SEQ ID NO: 22, or an amino acid sequence having at least 90 % or 95 % sequence identity thereto. In another embodiment, the binding protein based on the T3 TCR comprises an antigen binding unit comprising a β-chain variable domain comprising or consisting of the amino acid sequence set forth in SEQ ID NO: 23, or an amino acid sequence having at least 90 % or 95 % sequence identity thereto. In a particular embodiment, the binding protein based on the T3 TCR comprises an antigen binding unit comprising an α-chain variable domain comprising an amino acid sequence comprising or consisting of the amino acid sequence set forth in SEQ ID NO: 22, or an amino acid sequence having at least 90 % or 95 % sequence identity thereto; and a β-chain variable domain comprising or consisting of the amino acid sequence set forth in SEQ ID NO: 23, or an amino acid sequence having at least 90 % or 95 % sequence identity thereto.

When the binding protein comprises an α-chain variable domain comprising a variant of SEQ ID NO: 22 (i.e. an amino acid sequence with at least 90 %, but less than 100 %, sequence identity to SEQ ID NO: 22), the VαCDR sequences are nonetheless as defined above (i.e. VαCDR1, VαCDR2 and VαCDR3 comprise or consist of the amino acid sequences set forth in SEQ ID NOs: 16, 17 and 18, respectively). Thus any sequence modifications are made within the framework regions of the variable domain. When the binding protein comprises a β-chain variable domain comprising a variant of SEQ ID NO: 23 (i.e. an amino acid sequence with at least 90 %, but less than 100 %, sequence identity to SEQ ID NO: 23), the VβCDR sequences are nonetheless as defined above (i.e. VβCDR1, VβCDR2 and VβCDR3 comprise or consist of the amino acid sequences set forth in SEQ ID NOs: 19, 20 and 21, respectively). Thus any sequence modifications are made within the framework regions of the variable domain.

The second aspect of the present disclosure provides a binding protein as described above based on the T1 TCR.

The α-chain and β-chain of the T1 TCR have variable domains comprising the amino acid sequences set forth in SEQ ID NO: 8 and SEQ ID NO: 9, respectively. In a particular embodiment, the binding protein based on the T1 TCR comprises an antigen binding unit comprising an α-chain variable domain comprising or consisting of the amino acid sequence set forth in SEQ ID NO: 8, or an amino acid sequence having at least 90 % or 95 % sequence identity thereto. In another embodiment, the binding protein based on the T1 TCR comprises an antigen binding unit comprising a β-chain variable domain comprising or consisting of the amino acid sequence set forth in SEQ ID NO: 9, or an amino acid sequence having at least 90 % or 95 % sequence identity thereto. In a particular embodiment, the binding protein based on the T1 TCR comprises an antigen binding unit comprising an α-chain variable domain comprising an amino acid sequence comprising or consisting of the amino acid sequence set forth in SEQ ID NO: 8, or an amino acid sequence having at least 90 % or 95 % sequence identity thereto; and a β-chain variable domain comprising or consisting of the amino acid sequence set forth in SEQ ID NO: 9, or an amino acid sequence having at least 90 % or 95 % sequence identity thereto.

When the binding protein comprises an α-chain variable domain comprising a variant of SEQ ID NO: 8 (i.e. an amino acid sequence with at least 90 %, but less than 100 %, sequence identity to SEQ ID NO: 8), the VαCDR sequences are nonetheless as defined above (i.e. VαCDR1, VαCDR2 and VαCDR3 comprise or consist of the amino acid sequences set forth in SEQ ID NOs: 2, 3 and 4, respectively). Thus any sequence modifications are made within the framework regions of the variable domain. When the binding protein comprises a β-chain variable domain comprising a variant of SEQ ID NO: 9 (i.e. an amino acid sequence with at least 90 %, but less than 100 %, sequence identity to SEQ ID NO: 9), the VβCDR sequences are nonetheless as defined above (i.e. VβCDR1, VβCDR2 and VβCDR3 comprise or consist of the amino acid sequences set forth in SEQ ID NOs: 5, 6 and 7, respectively). Thus any sequence modifications are made within the framework regions of the variable domain.

A "binding protein", as defined herein, is a protein which binds (or recognises) a target molecule. The binding proteins provided herein are capable of specific binding to MHC-I molecules comprising peptides derived from human TdT, as described above. By "specific binding" is meant that the binding proteins bind specifically to their particular molecular partners (the molecular partner of the binding protein derived from the T3 TCR being an HLA-A2 molecule presenting the TdT peptide of SEQ ID NO: 15; and the molecular partner of the binding protein derived from the T1 TCR being an HLA-A2 molecule presenting the TdT peptide of SEQ ID NO: 1). Specific binding to a target may be distinguished from off-target or non-specific binding. For example, the binding proteins bind their molecular partners with a higher affinity than they bind other molecules (or at least most other molecules). The binding of a binding protein to a target may be measured by any suitable method known in the art, e.g. surface plasmon resonance (SPR).

In particular, the binding proteins bind their molecular partners (as defined above) with a higher affinity than with which they bind MHC-peptide complexes comprising peptides derived from proteins other than TdT. The binding proteins may display minimal or no binding to non-cognate MHC-peptide complexes, in particular to MHC-peptide complexes comprising a peptide derived from a protein other than TdT. The binding proteins provided herein may specifically bind their molecular partners under physiological conditions, i.e. the conditions that would be found within the body of a host animal, in particular the body of a human. In particular, the binding proteins may specifically bind their molecular partners in the conditions found within a human tumour.

As detailed above, the T3 TCR specifically binds an MHC-I complex comprising HLA-A2 and the peptide of SEQ ID NO: 15, and the T1 TCR specifically binds an MHC-I complex comprising HLA-A2 and the peptide of SEQ ID NO: 1. Notably, although the peptides of SEQ ID NO: 1 and SEQ ID NO: 15 are very similar (as detailed above, the peptides differ only by a two amino acid extension at the C-terminus of SEQ ID NO: 15), as shown in the Examples below the T3 and T1 TCRs do not demonstrate cross-reactivity with their respective binding partners.

As detailed above, the binding proteins provided herein comprise an antigen binding unit comprising an α-chain variable domain and a β-chain variable domain. An "α-chain variable domain" as defined herein is a variable domain from an α-chain of a TCR (particularly a human TCR), or a polypeptide based thereon. Similarly, a "β-chain variable domain" as defined herein is a variable domain from a β-chain of a TCR (particularly a human TCR), or a polypeptide based thereon. Polypeptides based on a variable domain of an α- or β-chain of a TCR include e.g. variants of the variable domain comprising sequence modifications relative to the natural variable domain.

An "antigen binding unit" as referred to herein is simply the domain of a binding protein that is formed from the α- and β-chain variable domains and binds the target MHC-peptide complex.

Thus the minimal binding protein of the present disclosure is an antigen binding unit comprising an α-chain variable domain and a β-chain variable domain as indicated in **Figure 1B**.

The α-chain variable domain may be covalently connected to the β-chain variable domain, e.g. by a peptide linker, to form antigen binding units as indicated in **Figure 1C** and **Figure 1D**. Such antigen binding units and proteins comprising them are disclosed in WO 91/18019 (Squibb & Sons, Dana Farber Cancer Institute), WO 96/18105 (Harvard) and WO 2000/031239 (Yeda R&D). Suitable peptide linkers may comprise e.g. from 1 to 20 amino acids, such as for example 1, 2, 3 or 4 amino acids, 5, 10 or 15 amino acids, or other intermediate numbers in the range 1 to 20 as convenient. The peptide linker may be formed from any generally convenient amino acid residues, such as glycine and/or serine. One example of a suitable linker is Gly₄Ser. Multimers of such linkers may be used, such as for example a dimer, a trimer, a tetramer or a pentamer, e.g. (Gly₄Ser)₂, (Gly₄Ser)₃, (Gly₄Ser)₄ or (Gly₄Ser)₅. A peptide linker may connect the C-terminus of the α-chain variable domain to the N-terminus of the β-chain variable domain, or the N-terminus of the α-chain variable domain to the C-terminus of the β-chain variable domain.

A binding protein composed of an α-chain variable domain linked to a β-chain variable domain by a polypeptide linker (as described above) is referred to herein as a TCR-scFv (by analogy to standard scFvs derived from the variable domains of antibodies). TCR-scFvs are minimal soluble TCR species, and are schematically shown in **Figure 1C**. In an embodiment the binding protein is a TCR-scFv. Uses for TCR-scFvs are discussed further below.

In another embodiment, the binding proteins provided herein comprise a first chain, comprising the α-chain variable domain; and a second chain, comprising the β-chain variable domain. That is to say, the α-chain variable domain and β-chain variable domain may be located within separate polypeptide chains (that associate with each other to form the binding proteins). The first and second chains thus correspond to the α and β chains of a classical full-length αβ TCR, but need not comprise all the domains which are naturally present in a full-length α or β chain. In an embodiment, the first and second chains may comprise all the domains of an α or β chain respectively, i.e. a variable domain, a constant domain, a transmembrane domain and a cytoplasmic domain (endodomain). In other embodiments, the first and second chains may comprise a variable domain and a constant domain. In other words, the first chain may comprise an α-chain variable domain and an α-chain constant domain. The second chain may comprise a β-chain variable domain and a β-chain constant domain. In still other embodiments the chains may further comprise a transmembrane domain, or a transmembrane domain and a cytoplasmic domain. Thus, the first and second chains may correspond to, or may represent, (or indeed may be referred to as) a full-length or a truncated α and β chain respectively. By "full length" it is meant that the chain comprises all the domains of an α or β chain. In other words, a full-length chain comprises a variable domain, a constant domain, a transmembrane domain, and a cytoplasmic domain. Whilst the transmembrane or cytoplasmic domains may be obtained or derived from the α or β-chain as appropriate, they may alternatively be obtained or derived from any other protein. Thus, a full-length α or β chain may be a natural or a synthetic or modified protein. By "truncated" it is meant that the chain may be missing all or part of any one or more of the constant domain, the transmembrane domain, or the cytoplasmic domain. Accordingly, in a full-length or truncated α or β chain according to the disclosure herein, whilst the variable and constant domains may be TCR α or β chain domains, the transmembrane and cytoplasmic domains are not limited to those obtained or derived from a TCR α or β chain. The term "first chain" as used broadly herein means a polypeptide chain which contains at least a variable domain obtained or derived from an a-chain, and the term "second chain" as used broadly herein means a polypeptide chain which contains at least a variable domain obtained or derived from a β-chain. The first chain may thus be viewed as an "α-based chain" and the second chain may be viewed as a "β-based chain".

In an embodiment the separate polypeptide chains may nonetheless be covalently joined (e.g. by one or more disulphide bonds formed between the side chains of cysteine residues). For instance, it is known that the extracellular constant domains of natural αβTCRs form an interchain disulphide-bridge, which is believed to stabilise the heterodimer. The interchain disulphide-bridge is depicted in **Figure 1A** as a solid vertical bar connecting the constant domains (C).

Thus, as noted above, the first chain (i.e. full-length or truncated α-chain) and/or the second chain (i.e. full-length or truncated β-chain) may comprise an extracellular constant domain. Without being bound by theory, interactions between the α-chain constant domain and the β-chain constant domain may stabilise the antigen binding unit. In one embodiment, the first chain comprises an extracellular α-chain constant domain (located C-terminal to the variable domain, for example directly C-terminal such that the N-terminal amino acid of the constant domain is joined by a peptide bond to the C-terminal amino acid of the variable domain). In another embodiment, the second chain comprises an extracellular β-chain constant domain (located C-terminal to the variable domain, for example directly C-terminal to the variable domain). In an embodiment, both the first chain and the second chain comprise an extracellular constant domain.

Any suitable extracellular constant domains may be used. The constant domains may be human TCR constant domains, or TCR constant domains from another animal, for instance the constant domains may be murine constant domains. Alternatively the constant domains may be synthetic constant domains, or constant domains derived from a synthetic protein. When the first and second chains both comprise constant domains, both constant domains may be from the same species or different species. The human α-chain and β-chain types 1 and 2 extracellular constant domain sequences are set forth in SEQ ID NOs: 28-30, respectively. In an embodiment, the extracellular α-chain constant domain comprises the amino acid sequence set forth in SEQ ID NO: 28, or an amino acid sequence having at least 90 % or 95 % sequence identity thereto; and/or the extracellular β-chain constant domain comprises the amino acid sequence set forth in SEQ ID NO: 29 or SEQ ID NO: 30, or an amino acid sequence having at least 90 % or 95 % sequence identity thereto.

The murine α-chain and β-chain types 1 and 2 extracellular constant domain sequences are set forth in SEQ ID NOs: 31-33, respectively. In a particular embodiment, the extracellular α-chain constant domain comprises the amino acid sequence set forth in SEQ ID NO: 31, or an amino acid sequence having at least 90 % or 95 % sequence identity thereto; and/or the extracellular β-chain constant domain comprises the amino acid sequence set forth in SEQ ID NO: 32 or SEQ ID NO: 33, or an amino acid sequence having at least 90 % or 95 % sequence identity thereto.

It is also known that an additional cysteine bridge may be introduced by insertion or by substituting a suitable amino acid residue on each chain with a cysteine residue. **Figures 1E** and **1F** show binding proteins with additional cysteine bridges (depicted as solid vertical bars connecting the constant domains (C)). The additional cysteine residues should be introduced at locations such that formation of a disulphide bond between the introduced residues leads to the formation of a functional complex in which the variable regions are correctly located and orientated with respect to each other, e.g. as disclosed by Boulter, J.M. et al. (Protein Eng. Des. Sel. 16(9): 707-711, 2003).

Known suitable locations in human TCR chain constant regions for the introduction of cysteine residues are disclosed in Cohen et al. (Cancer Research 67(8):3898-903, 2007) and WO 2019/166463. In the human TCR α-chain constant region of SEQ ID NO: 28, an additional cysteine residue may be introduced by substitution of the threonine residue at position 49 for a cysteine residue. The modified human α-chain extracellular constant domain obtained by this Thr49Cys substitution has the amino acid sequence set forth in SEQ ID NO: 34. In the human TCR β-chain type 1 constant region of SEQ ID NO: 29 or type 2 constant region of SEQ ID NO: 30, an additional cysteine residue may be introduced by substitution of the serine residue at position 57 for a cysteine residue. The modified human β-chain extracellular constant domains obtained by these Ser57Cys substitutions have the amino acid sequences set forth in SEQ ID NOs: 35 and 36, respectively. Thus in an embodiment the first chain comprises an extracellular α-chain constant domain comprising the amino acid sequence set forth in SEQ ID NO: 34, or an amino acid sequence having at least 90 % or 95 % sequence identity thereto; and the second chain comprises an extracellular β-chain constant domain comprising the amino acid sequence set forth in SEQ ID NO: 35 or 36, or an amino acid sequence having at least 90 % or 95 % sequence identity thereto.

As detailed above, the extracellular constant domains of SEQ ID NOs: 34-36 have been modified in order to form two interchain disulphide bonds. Accordingly, where a binding protein as provided herein comprises a variant of any one of these sequences as an extracellular constant domain (i.e. an amino acid sequence having at least 90 %, but less than 100 %, sequence identity to any one of SEQ ID NOs: 34-36), the cysteine residues involved in disulphide bond formation may be retained (i.e. are not substituted or deleted). In the α-chain extracellular domain of SEQ ID NO: 34, the cysteine residues involved in disulphide bond formation are Cys49 and Cys96. In the β-chain extracellular domains of SEQ ID NOs: 35 and 36, the cysteine residues involved in disulphide bond formation are Cys57 and Cys131.

In murine TCR chain extracellular constant domains, the positions equivalent to those described above in the human TCR constant domains are suitable for introduction of additional cysteine residues. Thus in the murine TCR α-chain constant region of SEQ ID NO: 31, an additional cysteine residue may be introduced by substitution of the threonine residue at position 49 for a cysteine residue. The modified murine α-chain extracellular constant domain obtained by this Thr49Cys substitution has the amino acid sequence set forth in SEQ ID NO: 10. In the murine TCR β-chain type 1 constant region of SEQ ID NO: 32 or type 2 constant region of SEQ ID NO: 33, an additional cysteine residue may be introduced by substitution of the serine residue at position 57 for a cysteine residue. The modified murine β-chain extracellular constant domains obtained by these Ser57Cys substitutions have the amino acid sequences set forth in SEQ ID NOs: 37 and 11, respectively. Thus in a particular embodiment the first chain comprises an extracellular α-chain constant domain comprising the amino acid sequence set forth in SEQ ID NO: 10, or an amino acid sequence having at least 90 % or 95 % sequence identity thereto; and the second chain comprises an extracellular β-chain constant domain comprising the amino acid sequence set forth in SEQ ID NO: 11 or 37, or an amino acid sequence having at least 90 % or 95 % sequence identity thereto.

As for the modified human sequences above, in a variant of any one of SEQ ID NOs: 10, 11 and 37 (i.e. an amino acid sequence having at least 90 %, but less than 100 %, sequence identity to any one of SEQ ID NOs: 10, 11 or 37), the cysteine residues involved in disulphide bond formation may be retained (i.e. are not substituted or deleted). In the α-chain extracellular domain of SEQ ID NO: 10, the cysteine residues involved in disulphide bond formation are Cys49 and Cys92. In the β-chain extracellular domains of SEQ ID NOs: 35 and 36, the cysteine residues involved in disulphide bond formation are Cys57 and Cys127.

In the T1 and T3 TCRs, the α-chain comprises an extracellular α-chain constant domain comprising the amino acid sequence set forth in SEQ ID NO: 10, and the β-chain comprises an extracellular β-chain constant domain comprising the amino acid sequence set forth in SEQ ID NO: 11. Thus in a particular embodiment, the binding protein comprises a first chain comprising an extracellular α-chain constant domain comprising or consisting of the amino acid sequence set forth in SEQ ID NO: 10, or an amino acid sequence having at least 90 % or 95 % sequence identity thereto; and a second chain comprising an extracellular β-chain constant domain comprising or consisting of the amino acid sequence set forth in SEQ ID NO: 11, or an amino acid sequence having at least 90 % or 95 % sequence identity thereto.

A binding protein as provided herein comprising a first chain and a second chain each comprising a variable domain and an extracellular domain may be a soluble TCR (i.e. which is not embedded in the membrane), as shown in **Figure 1E** and previously disclosed in Walseng et al. (PLoS ONE 10(4): e0119559, 2015) and Walseng et al. (Scientific Reports 7: Article 10713, 2017). The term "soluble TCR" as used herein means a TCR molecule which comprises separate first and second chains but which is not membrane-bound. In other words, a soluble TCR does not comprise a TM domain or a cytoplasmic domain. A soluble TCR may in particular comprise first and second chains each consisting of extracellular domains only.

It is an essential aspect of a soluble TCR that the first and second chains are joined. If they are not joined, the chains will diffuse apart in solution and the TCR will function poorly, if at all. The chains may be joined covalently or non-covalently. One method by which the truncated α- and β-chains can be covalently joined is by one or more disulphide bonds between cysteine residues, as described above. An alternative method by which the truncated α- and β-chains of the soluble TCR may be joined is by non-covalent interactions. In one embodiment, leucine zippers are used to non-covalently join the chains. In this embodiment, both the truncated α- and β-chains comprise leucine zipper domains at the C-termini of their extracellular constant domains. Leucine zippers, and their sequences, are well-known in the art, and are reviewed in e.g. Busch & Sassone-Corsi (Trends in Genetics 6: 36-40, 1990). Combinations of covalent and non-covalent interactions may also be used to join the truncated α- and β-chains of the soluble TCR.

Uses of soluble TCRs (and scFv-TCRs, which are also soluble but do not fall within the definition of "soluble TCR" as used herein) are described further below. Soluble TCRs and scFv-TCRs may be encoded with an affinity tag, which may be used in purification of the soluble TCR or scFv-TCR following its synthesis. In the case of a soluble TCR, in an embodiment only one chain (i.e. the truncated α- or β-chain) is produced with an affinity tag. The affinity tag may be located at either terminus of the truncated α- or β-chain, for instance at the C-terminus. The affinity tag may be any suitable tag known to the skilled person, e.g. a FLAG-tag, a His-tag, an HA-tag, a Strep-tag, an S-tag, a Myc-tag, glutathione S-transferase (GST), maltose-binding protein (MBP), etc.

A linker and/or a protease cleavage site may be located between the binding protein sequence and the affinity tag. The inclusion of a protease cleavage site enables removal of the affinity tag from the binding protein following purification. Appropriate protease cleavage sites are well-known to the skilled person and include thrombin, factor Xa, enterokinase, human rhinovirus (HRV) 3C and tobacco etch virus (TEV) cleavage sites.

The first chain and/or the second chain may further comprise a transmembrane domain. In a first or second chain, the transmembrane domain is located C-terminal to the extracellular constant domain.

Any suitable transmembrane domain may be used. In a particular embodiment, the transmembrane domain is the transmembrane domain of a TCR chain. That is to say, when the first chain comprises a transmembrane domain, it may be a transmembrane domain of a TCR α-chain; and when the second chain comprises a transmembrane domain, it may be a transmembrane domain of a TCR β-chain. In one embodiment, the transmembrane domains are the transmembrane domains of human TCR chains. In another embodiment, the transmembrane domains are the transmembrane domains of murine TCR chains. In one embodiment, when both the first and second chains comprise TCR chain transmembrane domains, the TCR transmembrane domains both come from the same species.

The human TCR α-chain transmembrane domain has the amino acid sequence set forth in SEQ ID NO: 38, and thus in one embodiment the first chain comprises a transmembrane domain comprising the amino acid sequence set forth in SEQ ID NO: 38, or an amino acid sequence having at least 90 % or 95 % sequence identity thereto. The murine α-chain transmembrane domain has the amino acid sequence set forth in SEQ ID NO: 44, and thus in another embodiment the first chain comprises a transmembrane domain comprising the amino acid sequence set forth in SEQ ID NO: 44, or an amino acid sequence having at least 90 % or 95 % sequence identity thereto.

The human and murine TCR β-chains type 1 and 2 transmembrane domains all have the amino acid sequence set forth in SEQ ID NO: 39. Thus in a particular embodiment the second chain comprises a transmembrane domain comprising the amino acid sequence set forth in SEQ ID NO: 39, or an amino acid sequence having at least 90 % sequence identity thereto.

The T1 and T3 TCRs disclosed in the Examples both have an α-chain with the transmembrane domain of SEQ ID NO: 44 and a β-chain with the transmembrane domain of SEQ ID NO: 39. Thus in a particular embodiment, the first and second chains both comprise a transmembrane domain, and the transmembrane domain of the first chain of the binding proteins comprises the amino acid sequence set forth in SEQ ID NO: 44, or an amino acid sequence having at least 90 % or 95 % sequence identity thereto; and the transmembrane domain of the second chain of the binding proteins comprises the amino acid sequence set forth in SEQ ID NO: 39, or an amino acid sequence having at least 90 % sequence identity thereto.

Alternatively, and as further discussed below, the transmembrane domain may be based on or derived from the transmembrane domain of any other transmembrane protein. Typically it may be, or may be derived from, a transmembrane domain from CD8α, CD28, CD4, CD3ζ, CD45, CD9, CD16, CD22, CD33, CD64, CD80, CD86, CD134, CD137, or CD154, for instance from a human version of any one of these proteins. In one embodiment, the transmembrane domain may be, or may be derived from, a transmembrane domain from CD8α, CD28, CD4, or CD3ζ, for instance from human CD28, CD4, or CD3ζ.

In one embodiment the transmembrane domain is the transmembrane domain of human CD28, which has the amino acid sequence of SEQ ID NO: 40, i.e. the transmembrane domain may comprise the amino acid sequence set forth in SEQ ID NO: 40, or an amino acid sequence having at least 95 % sequence identity thereto.

The first chain and/or the second chain may further comprise a cytoplasmic domain. As is known in the art, in a native TCR the α- and β-chains comprise short cytoplasmic domains, located at the C-termini. Any suitable cytoplasmic domain may be used. In one embodiment the cytoplasmic domain is the cytoplasmic domain of a TCR chain. That is to say, when the first chain is an α-chain comprising a cytoplasmic domain, it may be a cytoplasmic domain of a TCR α-chain; and when the second chain is a β-chain comprising a cytoplasmic domain, it may be a cytoplasmic domain of a TCR β-chain.

In one embodiment, the cytoplasmic domains are the cytoplasmic domains of human TCR chains. In another embodiment the cytoplasmic domains are the cytoplasmic domains of murine TCR chains. When both the first and second chains comprise TCR chain cytoplasmic domains, the TCR cytoplasmic domains may both come from the same species.

The human and murine TCR α-chain cytoplasmic domains have the amino acid sequence set forth in SEQ ID NO: 41, and thus in one embodiment the first chain is an α-chain which comprises a cytoplasmic domain comprising the amino acid sequence set forth in SEQ ID NO: 41, or an amino acid sequence having at least 80 % sequence identity thereto. The human TCR β-chain type 1 cytoplasmic domain has the amino acid sequence set forth in SEQ ID NO: 42, and the human TCR β-chain type 2 cytoplasmic domain has the amino acid sequence set forth in SEQ ID NO: 43. Thus in one embodiment the second chain is a β-chain which comprises a cytoplasmic domain comprising the amino acid sequence set forth in SEQ ID NO: 42 or SEQ ID NO: 43, or an amino acid sequence having at least 90 % or 95 % sequence identity thereto.

The murine TCR β-chain type 1 cytoplasmic domain has the amino acid sequence set forth in SEQ ID NO: 46, and the murine TCR β-chain type 2 cytoplasmic domain has the amino acid sequence set forth in SEQ ID NO: 47. Thus in one embodiment the second chain is a β-chain which comprises a cytoplasmic domain comprising the amino acid sequence set forth in SEQ ID NO: 46 or SEQ ID NO: 47, or an amino acid sequence having at least 90 % sequence identity thereto.

The T1 and T3 TCRs both have an α-chain with the cytoplasmic domain of SEQ ID NO: 41 and a β-chain with the cytoplasmic domain of SEQ ID NO: 47. In a particular embodiment, the first chain and the second chain both comprise a cytoplasmic domain (i.e. they are full-length α and β chains), and the cytoplasmic domain of the α-chain comprises the amino acid sequence set forth in SEQ ID NO: 41, or an amino acid sequence having at least 80 % sequence identity thereto; and the cytoplasmic domain of the β-chain comprises the amino acid sequence set forth in SEQ ID NO: 47, or an amino acid sequence having at least 90 % sequence identity thereto.

In another embodiment, the first and/or second chain (generally either the first chain or the second chain) comprises a cytoplasmic domain which comprises an intracellular signalling domain. The term "intracellular signalling domain" refers herein to a domain of the binding protein chain that participates in transducing the message of effective receptor binding to a target antigen-MHC complex into the interior of an immune effector cell expressing the receptor, to elicit effector cell function, e.g. activation, cytokine production, proliferation and/or cytotoxic activity, including the release of cytotoxic factors to the bound target cell, or other cellular responses elicited by binding of the receptor to its target.

Examples of intracellular signalling domains that may be used include those derived from CD3ζ, FcRy, FcRβ, CD3γ, CD3δ, CD3ε, CD5, CD22, CD79a, CD79b and CD66d. In some embodiments, the intracellular signalling domain is derived from CD3ζ or FcRγ, for instance human CD3ζ or FcRγ. In one embodiment the intracellular signalling domain is a human CD3ζ domain comprising the amino acid sequence set forth in SEQ ID NO: 48, or an amino acid sequence having at least 95 % sequence identity to SEQ ID NO: 48.

Additionally, to allow or to augment full activation of an immune effector cell expressing the receptor, the cytoplasmic domain may further comprise a co-stimulatory domain (in addition to the intracellular signalling domain). Thus, the intracellular signalling domain may initiate antigen-dependent primary activation (i.e. may be a primary cytoplasmic signalling sequence) and the co-stimulatory domain may act in an antigen-independent manner to provide a secondary or co-stimulatory signal (i.e. it may be a secondary cytoplasmic signalling sequence).

The term "co-stimulatory signalling domain" or "co-stimulatory domain", refers to the portion of the cytoplasmic domain comprising the intracellular domain of a co-stimulatory molecule. Co-stimulatory molecules are cell surface molecules other than antigen receptors or Fc receptors that provide a second signal required for efficient activation and function of an immune effector cell (e.g. a T cell) upon binding to an antigen. Examples of co-stimulatory domains that may be used herein include the intracellular domains of CD27, CD28, 4-IBB (CD137), OX40 (CD134), CD30, CD40, PD-1, ICOS (CD278), LFA-1, CD2, CD7, LIGHT, NKD2C and B7-H2, for instance the human versions thereof. The co-stimulatory domains may be used singly or in combination (i.e. one or more co-stimulatory domains may be included). The inclusion of one or more co-stimulatory signalling domains may enhance the efficacy and expansion of immune effector cells expressing the receptors provided herein. In an embodiment the co-stimulatory domain may be, or may include, the intracellular domain of human CD28 having the amino acid sequence of SEQ ID NO. 49, or an amino acid sequence having at least 95 % sequence identity to SEQ ID NO: 49.

The examples herein demonstrate the functionality of full-length T1 and T3 TCRs comprising an α chain and a β chain (the term "full-length TCR" is interchangeable herein with the term "TCR"). It is demonstrated that T cells modified to express these TCRs, which target peptides from the lymphoid-specific enzyme terminal deoxynucleotidyl transferase (TdT) in the context of HLA-A*02:01, specifically eliminate primary acute lymphoblastic leukaemia (ALL) cells of T and B cell origin, both *in vitro* and in a mouse model of disseminated ALL. In contrast, the normal, mature T and B cell repertoires and non-lineage committed haematopoietic progenitor cells, lacking TdT expression, are spared. The extensive mapping of TCR-reactivity described in the Examples did not identify cross-reactivity with any naturally occurring peptide in the human proteome, indicating very high peptide specificity.

As described above and in the Examples, the T1 and T3 TCRs comprise human TCR variable domains and murine TCR extracellular constant domains, transmembrane domains and cytoplasmic domains. Thus in a particular embodiment, the binding protein provided herein is a TCR, having a structure as exemplified in **Figure 1A****.** In this embodiment, the TCR thus comprises an α-chain and a β-chain, the α-chain comprising a TCR α-chain variable domain, a TCR α-chain extracellular constant domain, a TCR α-chain transmembrane domain and a TCR α-chain cytoplasmic domain; and the β-chain comprising a TCR β-chain variable domain, a TCR β-chain extracellular constant domain, a TCR β-chain transmembrane domain and a TCR β-chain cytoplasmic domain.

The various domains may have the sequences set out above. In particular, the TCR may comprise an α-chain and a β-chain, each of which comprises a human variable domain and murine extracellular constant, transmembrane and cytoplasmic domains.

In a particular embodiment, the TCR is a T3 TCR, comprising:
(i) an α-chain comprising, from N-terminus to C-terminus: a variable domain comprising the amino acid sequence set forth in SEQ ID NO: 22, or an amino acid sequence having at least 90 % or 95 % sequence identity thereto; an extracellular constant domain comprising the amino acid sequence set forth in SEQ ID NO: 10, or an amino acid sequence having at least 90 % or 95 % sequence identity thereto; a transmembrane domain comprising the amino acid sequence set forth in SEQ ID NO: 44, or an amino acid sequence having at least 90 % or 95 % sequence identity thereto; and a cytoplasmic domain comprising the amino acid sequence set forth in SEQ ID NO: 41, or an amino acid sequence having at least 80 % sequence identity thereto; and
(ii) a β-chain comprising, from N-terminus to C-terminus: a variable domain comprising the amino acid sequence set forth in SEQ ID NO: 23, or an amino acid sequence having at least 90 % or 95 % sequence identity thereto; an extracellular constant domain comprising the amino acid sequence set forth in SEQ ID NO: 11, or an amino acid sequence having at least 90 % or 95 % sequence identity thereto; a transmembrane domain comprising the amino acid sequence set forth in SEQ ID NO: 39, or an amino acid sequence having at least 90 % sequence identity thereto; and a cytoplasmic domain comprising the amino acid sequence set forth in SEQ ID NO: 47, or an amino acid sequence having at least 90 % sequence identity thereto.

The full-length mature T3 TCR α-chain has the amino acid sequence set forth in SEQ ID NO: 24. Thus in one embodiment, the binding protein comprises an α-chain comprising the amino acid sequence set forth in SEQ ID NO: 24, or an amino acid sequence having at least 90 % or 95 % sequence identity thereto. The full-length mature T3 TCR β-chain has the amino acid sequence set forth in SEQ ID NO: 25. Thus in one embodiment, the binding protein comprises a β-chain comprising the amino acid sequence set forth in SEQ ID NO: 25, or an amino acid sequence having at least 90 % or 95 % sequence identity thereto.

In a particular embodiment, the TCR provided herein is a T3 TCR comprising:
(i) an α-chain comprising the amino acid sequence set forth in SEQ ID NO: 24, or an amino acid sequence having at least 90 % or 95 % sequence identity thereto; and
(ii) a β-chain comprising the amino acid sequence set forth in SEQ ID NO: 25, or an amino acid sequence having at least 90 % or 95 % sequence identity thereto.

In another particular embodiment, the TCR is a T1 TCR, comprising:
(i) an α-chain comprising, from N-terminus to C-terminus: a variable domain comprising the amino acid sequence set forth in SEQ ID NO: 8, or an amino acid sequence having at least 90 % or 95 % sequence identity thereto; an extracellular constant domain comprising the amino acid sequence set forth in SEQ ID NO: 10, or an amino acid sequence having at least 90 % or 95 % sequence identity thereto; a transmembrane domain comprising the amino acid sequence set forth in SEQ ID NO: 44, or an amino acid sequence having at least 90 % or 95 % sequence identity thereto; and a cytoplasmic domain comprising the amino acid sequence set forth in SEQ ID NO: 41, or an amino acid sequence having at least 80 % sequence identity thereto; and
(ii) a β-chain comprising, from N-terminus to C-terminus: a variable domain comprising the amino acid sequence set forth in SEQ ID NO: 9, or an amino acid sequence having at least 90 % or 95 % sequence identity thereto; an extracellular constant domain comprising the amino acid sequence set forth in SEQ ID NO: 11, or an amino acid sequence having at least 90 % or 95 % sequence identity thereto; a transmembrane domain comprising the amino acid sequence set forth in SEQ ID NO: 39, or an amino acid sequence having at least 90 % sequence identity thereto; and a cytoplasmic domain comprising the amino acid sequence set forth in SEQ ID NO: 47, or an amino acid sequence having at least 90 % sequence identity thereto.

The full-length mature T1 TCR α-chain has the amino acid sequence set forth in SEQ ID NO: 12. Thus in one embodiment, the binding protein comprises an α-chain comprising the amino acid sequence set forth in SEQ ID NO: 12, or an amino acid sequence having at least 90 % or 95 % sequence identity thereto. The full-length mature T1 TCR β-chain has the amino acid sequence set forth in SEQ ID NO: 13. Thus in one embodiment, the binding protein comprises a β-chain comprising the amino acid sequence set forth in SEQ ID NO: 13, or an amino acid sequence having at least 90 % or 95 % sequence identity thereto.

In a particular embodiment, the TCR provided herein is a T1 TCR comprising:
(i) an α-chain comprising the amino acid sequence set forth in SEQ ID NO: 12, or an amino acid sequence having at least 90 % or 95 % sequence identity thereto; and
(ii) a β-chain comprising the amino acid sequence set forth in SEQ ID NO: 13, or an amino acid sequence having at least 90 % or 95 % sequence identity thereto.

As noted above, the cytoplasmic domain of the binding protein provided herein may comprise an intracellular signalling domain (and optionally a co-stimulatory domain) rather than a TCR cytoplasmic domain. Such alternative cytoplasmic domains may be used in chimeric receptors.

An exemplary chimeric receptor is a chimeric TCR, the basic structure of which is shown in **Figure 1D**. Chimeric TCRs have been previously described in WO 2000/031239 and WO 2019/166463. A chimeric TCR comprises a single polypeptide chain, comprising (from N-terminus to C-terminus) a single-chain TCR variable region (scFv-TCR), an extracellular constant domain, a transmembrane domain and an intracellular signalling domain.

In a chimeric TCR as provided herein the scFv-TCR is as described above. the extracellular constant domain may be a constant domain of an α-chain or a constant domain of a β-chain. Any suitable extracellular constant domain of a TCR α- or β-chain may be used, e.g. a human or murine extracellular constant domain of a TCR α- or β-chain, as described above. A chimeric TCR comprises only a single extracellular constant domain of a TCR chain. A chimeric TCR as provided herein may comprise any suitable transmembrane domain, such as those described above. The transmembrane domain of the chimeric TCR may be a transmembrane domain of a TCR chain, as described above, or may be a transmembrane domain of a non-TCR protein, as described above. The cytoplasmic domain comprises an intracellular signalling domain, as described above. The cytoplasmic domain may further comprise a co-stimulatory domain, as described above.

In the context of a chimeric TCR, the transmembrane domain and the cytoplasmic domain together constitute the "signalling tail". In a signalling tail comprising both an intracellular signalling domain and a co-stimulatory domain, the intracellular signalling and co-stimulatory domains may be linked in any order in tandem to the C-terminus of the transmembrane domain. In one embodiment, the signalling tail comprises, in the following order N-terminal to C-terminal, the CD28 transmembrane domain, the CD28 intracellular domain and the CD3ζ intracellular domain.

Another exemplary chimeric receptor is a TCR-CAR, the basic structure of which is shown in **Figure 1F**. TCR-CARs were first described in Walseng *et al.,* 2017 (*supra*). A TCR-CAR is based on the same rationale as a standard CAR, but the scFv of a standard CAR is substituted with a soluble TCR (as described above). This provides a construct with the functional potential of a CAR, but with the substrate breadth of a TCR (i.e. they may be directed against any peptide resulting from cellular protein degradation).

A TCR-CAR thus comprises a soluble TCR as described above, but rather than being a soluble protein, one chain of the soluble TCR construct comprises a signalling tail, as described above in the context of a chimeric TCR. The signalling tail may be located at the C-terminus of either the first (i.e. truncated α-) chain or the second (i.e. truncated β-) chain (C-terminal to the extracellular constant domain).

Soluble binding proteins provided herein (i.e. binding proteins lacking a transmembrane domain, e.g. TCR-scFvs and soluble TCRs, as described above) may be synthesised using a protein expression system, such as a cellular expression system using prokaryotic (e.g. bacterial) cells or eukaryotic (e.g. yeast, fungus, insect or mammalian) cells. An alternative protein expression system is a cell-free, *in vitro* expression system, in which a DNA sequence encoding the binding protein is transcribed into mRNA, and the mRNA translated into a protein, *in vitro.* Cell-free expression system kits are widely available, and can be purchased from e.g. Thermo Fisher Scientific (USA). Alternatively, binding proteins may be chemically synthesised in a non-biological system, e.g. by liquid-phase or solid-phase synthesis

Following synthesis, the specific binding molecule is isolated and purified, using techniques known in the art. For instance, the binding protein may be produced using a host eukaryotic cell. The binding protein may be expressed such that it comprises a leader sequence which directs it for export (as discussed below). In this case the binding protein is exported from the production cell into the culture medium. The culture medium is then separated from the production cell, e.g. by centrifugation. The binding protein may then be purified, e.g. by affinity chromatography (if the binding protein comprises an affinity tag as discussed above). If a protease cleavage site is present between the tag and the mature binding protein, the tag may be cleaved using the appropriate protease, following purification of the binding protein.

Soluble binding proteins provided herein (i.e. binding proteins lacking a transmembrane domain, e.g. TCR-scFvs and soluble TCRs) may be linked or conjugated to a therapeutic or diagnostic agent, or a carrier which comprises or contains a therapeutic or diagnostic agent. A therapeutic agent is an agent used in therapy. By therapy is meant the treatment or prevention of a disease. The therapeutic agent may be an agent useful in the treatment of a neoplastic condition, particularly cancer.

The therapeutic agent may be a drug molecule, e.g. a toxin to kill a target cell. A suitable toxin is a toxin which, alone, is unable to enter, kill or otherwise disrupt a human cell but, when taken up by a human cell via a conjugated molecule, is able to exert its toxic effects. Such a toxin will thus only be taken up by, and exert its target effects on, a cell bound by a soluble binding protein carrying the toxin (such as a soluble TCR carrying the toxin), into which cell the soluble binding protein is taken up.

Suitable toxins include peptide toxins lacking a targeting domain. For instance, it may be a peptide toxin which natively lacks a targeting domain, or it may be a peptide toxin modified relative to its native form to remove its targeting domain. Examples of such toxins include saporin and gelonin, which are ribosome-inactivating proteins (RIPs) of the same family as e.g. ricin, but which are unable to cross the plasma membrane of a cell. Similarly, the enzymatic domain (i.e. catalytic domain) of a cytotoxin of a pathogen may be used, such as the enzymatic domain of a bacterial cytotoxin, e.g. the enzymatic domain of diphtheria toxin, *Pseudomonas* exotoxin A or a Clostridial cytotoxin, e.g. TcsL of *Clostridium sordellii.*

The soluble binding protein may be encoded as a fusion protein, with a toxin located at its C-terminus (in the case of a soluble TCR, the toxin may be located at the C-terminus of either the truncated α- or β-chain). Alternatively, the toxin may be conjugated to the soluble binding protein using any suitable method known in the art (e.g. using a biotin/streptavidin system).

The therapeutic agent may be any other useful therapeutic agent, for instance a chemotherapy agent, or any other anti-cancer agent, or anti-viral agent or suchlike, as appropriate.

A diagnostic agent is an agent useful for diagnostic purposes. Such an agent may for instance be a tracer or a label, i.e. an agent which can be detected in order to follow its passage through a human body. A tracer or label may be detected by a scan, e.g. a PET scan or a CT scan. Many tracers and labels are known in the art, including radiolabels. Any suitable tracer or label may be used, including the common radioisotopes ¹¹C, ¹³N, ¹⁵O, ¹³F, ⁹⁹Tc and ¹²³I and ¹²⁵I. A diagnostic agent may be conjugated to a soluble binding protein (e.g. a soluble TCR) using any suitable labelling group, such as are known in the art, e.g. radiolabelled biotin.

A soluble binding protein (e.g. soluble TCR) conjugated to a therapeutic agent may be used in therapy. A soluble TCR conjugated to a diagnostic agent, or a carrier comprising a diagnostic agent, may be used in *in vivo* diagnostic methods.

Insoluble binding proteins as provided herein (i.e. binding proteins comprising transmembrane domains, e.g. TCRs etc.) are provided in the context of a cell expressing the binding protein. Such a cell may in particular be an immune effector cell, in which functional expression of the binding protein is achieved, as described below.

Also provided herein are recombinant nucleic acid molecules encoding the binding proteins and/or antigen binding units provided herein. The nucleic acid molecule provided herein may be an isolated nucleic acid molecule and may include DNA or RNA or chemical derivatives of DNA or RNA. The term "nucleic acid molecule" specifically includes single- and double-stranded forms of DNA and RNA. The nucleic acid (e.g. DNA or RNA) may be circular or linear. A "recombinant" nucleic acid molecule is a nucleic acid molecule synthesised using recombinant techniques, e.g. molecular cloning.

The recombinant nucleic acid molecule provided herein encodes a binding protein as provided herein. As detailed above, the binding protein may comprise two polypeptide chains (in particular a first chain (i.e. a full-length or truncated α-chain) and a second chain (i.e. a full-length or truncated β-chain)). A recombinant nucleic acid molecule encoding a binding protein comprising two polypeptide chains encodes both of the two chains (i.e. the first chain and the second chain are both encoded by the same recombinant nucleic acid molecule). In this instance the two chains may be encoded as two separate genes, each under the control of its own promoter such that the two chains are separately expressed. Alternatively, as discussed further below, the two chains may be encoded within a single polypeptide, by a single gene.

In one particular embodiment herein, the recombinant nucleic acid molecule comprises a cDNA molecule. In particular, the binding protein or chains thereof may be encoded by cDNA. By "cDNA" as used herein is meant cDNA in its true and original sense (i.e. DNA synthesised by reverse transcription of mRNA), DNA amplified from original cDNA, and also DNA that is equivalent to cDNA. DNA that is equivalent to cDNA is DNA that encodes a binding protein as provided herein (or a first or second chain thereof) and that lacks introns, such that it resembles a protein-coding sequence obtained by reverse transcription of mRNA. In one particular embodiment herein, the nucleic acid molecule encoding a binding protein as provided herein is codon-optimised, in particular the binding protein may be encoded by a codon-optimised cDNA sequence.

Methods for constructing nucleotide sequences as defined herein are well known in the art, and include e.g. conventional polymerase chain reaction (PCR) cloning techniques.

Methods for isolation of nucleic acid molecules are also well known in the art. For instance, DNA may be isolated using a suitable kit. Plasmid DNA may be isolated from bacteria using a Miniprep or Maxiprep kit according to the manufacturer's instructions. Such kits are available from e.g. Qiagen (Germany). Genomic DNA may be extracted from eukaryotic or prokaryotic cells using e.g. a QIAamp DNA Mini Kit (Qiagen) or a DNeasy kit (Qiagen) according to the manufacturer's instructions. Alternatively, traditional methods of phenol-chloroform extraction may be used to isolate DNA from cells of interest. RNA may be extracted from cells using a kit (e.g. an RNeasy Mini Kit, Qiagen) or by traditional methods of phenol-chloroform extraction in combination with DNase treatment. cDNA may be generated by reverse transcription of RNA, e.g. using a kit such as SuperScript First Strand Synthesis System (Thermo Fisher Scientific, USA).

The recombinant nucleic acid molecule provided herein may be provided within a recombinant construct comprising the recombinant nucleic acid molecule linked to a heterologous nucleic acid sequence. By "heterologous" as used herein is meant a nucleic acid sequence which is not natively linked to the nucleic acid molecule described herein, i.e. which is not linked to the nucleic acid molecule described herein in nature. The term "linked" as used herein with respect to the construct may simply mean that the nucleic acid molecule is directly joined to a heterologous nucleic acid sequence. In one embodiment, in the recombinant construct the nucleic acid molecule provided herein is operatively linked to a heterologous expression control sequence.

The term "expression control sequence" refers to nucleotide sequences located upstream of, within, or downstream of a coding sequence, and which influence transcription, RNA processing or stability, or translation of the associated coding sequence (i.e. which influence any aspect of expression of the encoded specific binding molecule). Expression control sequences include promoters, promoter elements such as a TATA box or a B recognition element, operators, enhancers, translation leader sequences, terminator sequences and suchlike.

In the construct, the nucleic acid molecule may be operatively linked to one or more heterologous expression control sequences. The nucleic acid molecule is typically operatively linked to at least a promoter. Suitable promoter sequences include the cytomegalovirus (CMV) promoter, for instance the human CMV (HCMV) promoter, the PGK promoter, the EF1a promoter, the constitutive simian virus 40 (SV40) early promoter, the mouse mammary tumour virus (MMTV) promoter, the HIV LTR promoter, the MoMuLV promoter, the avian leukaemia virus promoter, the EBV immediate early promoter, and the Rous sarcoma virus promoter. Human gene promoters may also be used, including, but not limited to, the actin promoter, the myosin promoter, the haemoglobin promoter, and the creatine kinase promoter. In certain embodiments inducible promoters may be used. These provide a molecular switch capable of turning expression of the nucleic acid molecule on or off. Examples of inducible promoters include, but are not limited to, a metallothionine promoter, a glucocorticoid promoter, a progesterone promoter, or a tetracycline promoter.

The term "operably linked" refers to the association of two or more nucleic acid molecules on a single nucleic acid fragment so that the function of one is affected by the other. For example, a promoter is operably linked to a coding sequence when it is capable of affecting the expression of that coding sequence (i.e. the coding sequence is under the transcriptional control of the promoter).

As detailed above, the recombinant nucleic acid molecule provided herein encodes a binding protein as provided herein. When the binding protein comprises two polypeptide chains (i.e. a full length or truncated α-chain and a full length or truncated β-chain), e.g. if the binding protein is a TCR, both polypeptide chains are encoded by the same nucleic acid molecule. In this case, equimolar expression of the first and second chains may be achieved by encoding the binding protein as a single polypeptide comprising the first chain linked to the second chain, e.g. by a linker (i.e. the binding protein may be encoded as a single polypeptide chain in which the first and second chains are separated by a linker). The linker may have any suitable amino acid sequence. Suitable linkers are known in the art. The linker may be of any suitable length, e.g. it may be 1-30 amino acids long, for instance 1-25 or 1-20 amino acids long. The linker may be cleavable, allowing separation of the first and second polypeptides. If the first and second polypeptides cannot be separated, the two chains may not be able to adopt the correct conformations required for formation of the antigen-binding site from the variable regions of the first and second chains. The skilled person is able to select an appropriate linker. The linker may comprise a protease cleavage site to allow specific, post-translational cleavage of the linker and thus separation of the first and second polypeptides. Appropriate protease cleavage sites are well-known to the skilled person and include thrombin, factor Xa, enterokinase, human rhinovirus (HRV) 3C and tobacco etch virus (TEV) cleavage sites.

In a particular embodiment, the linker is self-splicing. A self-splicing linker is able to catalyse its own cleavage, thus separating the first and second polypeptides without the requirement of an active cleavage step. No stimulation or induction is required for the self-splicing reaction to occur. The cleavage reaction may completely excise the linker from the single chain specific binding molecule; alternatively the linker, or a part of the linker, may remain attached to one or both of the resultant separate polypeptide chains. The self-splicing reaction catalysed by the linker may occur post-translationally (i.e. it may be an autocatalytic proteolysis reaction), or it may occur co-translationally. Co-translational splicing can occur by preventing the formation of a peptide bond within the linker or between the linker and one of the polypeptide chains on either side of it.

A self-splicing linker may utilise a ribosomal skipping sequence. Such ribosomal skipping sequences are well-known to skilled persons. A suitable self-splicing linker is one derived from a picornavirus self-cleaving 2A peptide. 2A peptides are approximately 20-25 amino acids long and end with the conserved sequence motif Asp-Val/Ile-Glu-X-Asn-Pro-Gly-Pro (SEQ ID NO: 50). 2A peptides undergo co-translational self-splicing, by preventing the formation of a peptide bond between the conserved glycine reside in the penultimate position within the linker and the C-terminal proline residue, resulting effectively in cleavage of the protein between these two amino acids. After cleavage, the 2A peptide (with the exception of the C-terminal proline) remains attached to the C-terminus of the upstream protein; the final proline residue remains attached to the N-terminus of the downstream protein. 2A peptides are described in Lewis et al., 2015 (J Neurosci Methods 256: 22-29).

The Examples herein demonstrate expression of a TCR from a vector encoding the α-chain and β-chain separated by a porcine teschovirus-derived 2A sequence of SEQ ID NO: 26. A nucleic acid molecule provided herein may thus encode a binding protein as provided herein, wherein the binding protein is encoded in the form of a single polypeptide comprising a first chain (e.g. an α-chain) linked to a second chain (e.g. β-chain) by a self-splicing 2A peptide. The 2A peptide linker may have any suitable sequence, such that it has self-splicing activity. Several 2A peptide sequences are known, any of which may be used according to the present disclosure, e.g. 2A sequences disclosed in Wang et al., 2015 (Scientific Reports 5: Article No. 16273) and WO 2019/166463. So long as the linker includes the 2A peptide motif of SEQ ID NO: 50 at its C-terminus, essentially any sequence may be used. In one embodiment, the self-splicing linker is a 2A peptide comprising the amino acid sequence set forth in SEQ ID NO: 26, or an amino acid sequence having at least 50 %, 60 %, 70 %, 80 %, 90 % or 95 % sequence identity thereto.

The binding protein as provided herein (or, as relevant, the first chain and the second chain of the binding protein) may be encoded with an N-terminal leader sequence. Such leader sequences are believed to direct the binding protein (or chains thereof) to the cell membrane, either for export (in the case of a soluble binding protein) or insertion into the membrane (in the case of a binding protein comprising a transmembrane domain), and upon export of the protein or its insertion into the membrane, are trimmed off to yield the mature protein. An exemplary α-chain N-terminal leader sequence has the amino acid sequence set forth in SEQ ID NO: 51 and exemplary β-chain N-terminal leader sequences have the amino acid sequence set forth in SEQ ID NO: 52 and SEQ ID NO: 53 (which leader sequences are used in the Examples below). Specifically, SEQ ID NO: 52 is used as the leader sequence for the T1 TCR β-chain and SEQ ID NO: 53 is used as the leader sequence for the T3 TCR β-chain. Any suitable leader sequences may be used according to the present disclosure, but in one embodiment the first chain (e.g. α-chain) is encoded with a leader sequence comprising the amino acid sequence set forth in SEQ ID NO: 51, or an amino acid sequence having at least 90 % or 95 % sequence identity thereto; and the second chain (e.g. β-chain) is encoded with a leader sequence comprising the amino acid sequence set forth in SEQ ID NO: 52 or SEQ ID NO: 53, or an amino acid sequence having at least 90 % or 95 % sequence identity thereto.

In one embodiment, a T1 TCR-based binding protein may comprise a first chain (e.g. an α-chain) with a leader sequence of SEQ ID NO: 51, or an amino acid sequence having at least 90 % or 95 % sequence identity thereto; and a second chain (e.g. β-chain) with a leader sequence of SEQ ID NO: 52, or an amino acid sequence having at least 90 % sequence identity thereto. In another embodiment, a T3 TCR-based binding protein may comprise a first chain (e.g. an α-chain) with a leader sequence of SEQ ID NO: 51, or an amino acid sequence having at least 90 % or 95 % sequence identity thereto; and a second (e.g. a β-chain) with a leader sequence of SEQ ID NO: 53, or an amino acid sequence having at least 90 % or 95 % sequence identity thereto. When the binding protein comprises only a single polypeptide chain, any leader sequence may be used, e.g. a leader sequence from a TCR α-chain or a TCR β-chain, e.g. the leader sequence may comprise the amino acid sequence set forth in SEQ ID NO: 51, 52 or 53, or an amino acid sequence having at least 90 % or 95 % sequence identity thereto.

An exemplary single polypeptide comprising the T3 TCR chains has the amino acid sequence set forth in SEQ ID NO: 45. This polypeptide construct comprises, from N-terminus to C-terminus, the β-chain of SEQ ID NO: 25 with the N-terminal leader of SEQ ID NO: 53, the 2A peptide linker of SEQ ID NO: 26, and the α-chain of SEQ ID NO: 24 with the N-terminal leader of SEQ ID NO: 51. An exemplary single polypeptide comprising the T1 TCR chains has the amino acid sequence set forth in SEQ ID NO: 14. This polypeptide construct comprises, from N-terminus to C-terminus, the β-chain of SEQ ID NO: 13 with the N-terminal leader of SEQ ID NO: 52, the 2A peptide linker of SEQ ID NO: 26, and the α-chain of SEQ ID NO: 12 with the N-terminal leader of SEQ ID NO: 51. A DNA sequence encoding the T3 single chain TCR polypeptide is set forth in SEQ ID NO: 55; a DNA sequence encoding the T1 single chain TCR polypeptide is set forth in SEQ ID NO: 54.

The recombinant nucleic acid molecule or construct provided herein may be provided within a vector. The term "vector" as used herein refers to a vehicle into which the nucleic acid molecule or construct provided herein may be introduced (e.g. be covalently inserted) from which the specific binding molecule encoded by the nucleic acid molecule may be expressed and/or the nucleic acid molecule/construct cloned. The vector may accordingly be a cloning vector or an expression vector.

Examples of vectors include plasmids, autonomously replicating sequences and transposable elements. Additional exemplary vectors include, without limitation, phagemids, cosmids, artificial chromosomes such as a yeast artificial chromosome (YAC), a bacterial artificial chromosome (BAC) or a P1-derived artificial chromosome (PAC), bacteriophages such as lambda phage or M13 phage, and animal viruses, such as human viruses (i.e. viral vectors). Examples of categories of animal viruses useful as vectors include, without limitation, retroviruses (including lentiviruses), adenoviruses, adeno-associated viruses, herpesviruses (e.g. herpes simplex virus), poxviruses, baculoviruses, papillomaviruses and papovaviruses (e.g. SV40). Examples of expression vectors are pCI-neo vectors (Promega) for expression in mammalian cells and pLenti4/V5-DEST^{™} and pLenti6/V5-DEST^{™} for lentivirus-mediated gene transfer and expression in mammalian cells.

The vector may be a bacterial or prokaryotic vector (i.e. a vector for use, e.g. cloning or expression, in bacterial or prokaryotic cells) or a eukaryotic vector (i.e. a vector for use in mammalian cells), e.g. a mammalian vector. The nucleic acid molecule or construct provided herein may be produced in or introduced into a general purpose cloning vector, such as a bacterial cloning vector, e.g. an *Escherichia coli* cloning vector. Typically a cloning vector is a bacterial plasmid, e.g. an *E. coli* plasmid. Examples of such cloning vectors include pUC19 (available from e.g. New England Biolabs (NEB), USA), pBluescript vectors (Agilent, USA) and pCR TOPO^{®} vectors from Thermo Fisher Scientific.

The nucleic acid molecule or construct provided herein may be sub-cloned into an expression vector for expression of the specific binding molecule provided herein, such as a mammalian expression vector. Expression vectors can contain a variety of expression control sequences. The expression vector should have the necessary 5' upstream and 3' downstream regulatory elements such as promoter sequences (discussed above), including a TATA box, a Kozak sequence at the translation start site, and the 3' UTR AATAAA polyadenylation signal sequence to signal transcription termination, for efficient gene transcription and translation in its respective host cell.

In addition to control sequences that govern transcription and translation, vectors may contain additional nucleic acid sequences that serve other functions, including for example vector replication, selectable markers etc. Examples of selectable markers suitable for selection of bacterial host cells include antibiotic resistance genes, such as an ampicillin resistance gene (e.g. β-lactamase), a kanamycin resistance gene or a chloramphenicol resistance gene (e.g. chloramphenicol acetyl transferase). Selectable markers suitable for use in mammalian host cells include hygromycin-B phosphotransferase gene (hph) which confers resistance to hygromycin B, the amino glycoside phosphotransferase gene (neo or aph) from Tn5 which codes for resistance to the antibiotic G418, the dihydrofolate reductase (DHFR) gene, the adenosine deaminase gene (ADA), and the multi-drug resistance (MDR) gene. Such selectable markers allow the *in vitro* selection of cells carrying the vector.

The vector may comprise a marker which renders immune effector cells carrying the vector susceptible to negative selection *in vivo.* The inclusion of such a marker allows the selective destruction of immune effector cells carrying the vector in an individual to whom such cells have been administered, e.g. a patient treated using adoptive cell therapy. This may be important if e.g. the patient experiences severe side-effects to treatment. The negatively selectable phenotype may result from the insertion of a gene that confers sensitivity to an administered agent. Negatively selectable genes are known in the art, and include, *inter alia*: the Herpes simplex virus type I thymidine kinase (HSV-I TK) gene (Wigler et al., Cell 11 (1):223-232, 1977) which confers ganciclovir sensitivity; and bacterial cytosine deaminase, which confers 5-fluorocytosine sensitivity (Mullen et al., Proc. Natl. Acad. Sci. USA. 89:33-37 (1992)).

The vector may be a viral vector. A viral vector may be derived from a retrovirus, such as a lentivirus or a spumavirus/foamyvirus. As used herein, the term "viral vector" refers to a virus-derived particle which carries the nucleic acid molecule or construct provided herein, and is able to deliver the nucleic acid molecule/construct to a target cell. The viral vector can contain the nucleic acid molecule provided herein in place of nonessential viral genes, or in addition to the native viral genes. The vector can be utilised for the purpose of transferring DNA, RNA or other nucleic acids into cells either *in vitro* or *ex vivo.*

Numerous forms of viral vectors are known in the art, and any suitable viral vector may be used according to the present teaching, including both single-stranded and double-stranded RNA viral vectors and DNA viral vectors. Single-stranded viral vectors may be positive-sense or negative-sense viral vectors. In certain embodiments, the viral vector is a retroviral vector (i.e. a viral vector derived from a retrovirus), such as a lentiviral vector (i.e. a viral vector derived from a lentivirus). Because retroviral genomes integrate into the genome of an infected cell, a retroviral vector as described herein can be used to stably transduce a target cell, i.e. permanently alter the genetic make-up of a target cell.

The viral vector may be a self-inactivating vector or replication-deficient vector. Replication-deficient retroviral vectors can be obtained by e.g. modification (e.g. deletion or substitution) of the 3' LTR enhancer-promoter region, known as the U3 region, of the viral genome to prevent viral transcription beyond the first round of viral replication. Consequently, the vectors are capable of infecting and then integrating into the host genome only once, and cannot be passed further.

The retroviral vectors for use herein can be derived from any known retrovirus, e.g. Type C retroviruses, such as Moloney murine sarcoma virus (M-MSV), Harvey murine sarcoma virus (Ha-MuSV), mouse mammary tumour virus (MMTV), gibbon ape leukaemia virus (GaLV), feline leukaemia virus (FLV), spumaviruses, Friend virus, murine stem cell virus (MSCV) and Rous sarcoma virus (RSV); human T-cell leukaemia viruses such as HTLV-1 and HTLV-2; and the lentiviral family of retroviruses, such as the human immunodeficiency viruses HIV-1 and HIV-2, simian immunodeficiency virus (SIV), feline immunodeficiency virus (FIV), equine immunodeficiency virus (EIV), and other classes of retroviruses.

A retroviral packaging cell line (typically a mammalian cell line) may be used to produce viral vectors, which may then be used for transduction of T-cells. A packaging cell line may be used to produce a viral vector by transfection with one or more vectors (e.g. plasmids) which carry the necessary genes for viral particle assembly. Illustrative viral vectors are described in e.g. WO 2004/054512. An exemplary plasmid for the production of a retroviral vector is pMP71, as described in Wälchli et al. (PLoS ONE 6(11): e27930, 2011).

In another embodiment the vector is an mRNA vector. An mRNA vector is a positive-sense mRNA strand comprising the nucleic acid molecule or construct described above. An mRNA vector is a translatable mRNA strand which, upon delivery to a target cell, can bind a ribosome and initiate synthesis of the encoded protein. An mRNA vector is advantageous as it does not require nuclear entry or transcription in order to initiate production of its encoded protein, but can instead directly bind a cytoplasmic ribosome and initiate translation. Transfection of a target cell with an mRNA vector can thus be used for rapid production of the encoded specific binding molecule. RNA has only a limited half-life due to its inherent instability, and thus an mRNA vector may be used for transient transfection of a target cell.

An mRNA vector comprises the essential elements for translation, e.g. a 5' 7-methylguanylate cap for ribosomal recognition and a polyadenylate tail. An mRNA vector can be produced from an mRNA expression vector using a cellular or cell-free system according to methods known in the art. Suitable mRNA expression vectors include pClpA102 (Sæbøe-Larssen et al., J. Immunol. Methods 259: 191-203, 2002) and pClpA120-G (Wälchli *et al., supra*). Cell-free systems require a DNA template comprising the gene to be transcribed and a suitable promoter, and utilise an RNA polymerase, generally a phage RNA polymerase. Kits for the performance of *in vitro* transcription may be obtained from e.g. Thermo Fisher Scientific (e.g. the MEGAscript^{™} SP6 Transcription Kit).

Also provided herein is a kit comprising a first nucleic acid molecule encoding a first chain of a binding protein as provided herein and a second nucleic acid molecule encoding a second chain as provided herein. Thus the kit comprises a pair of nucleic acid molecules, one encoding a first chain and the other a second chain as described herein.

The first and second nucleic acid molecules may be as discussed above, e.g. they may be DNA or RNA, double- or single-stranded, linear or circular, etc. The first recombinant nucleic acid molecule and second nucleic acid molecule may be provided in the kit in the context of a first recombinant construct and a second recombinant construct (the first recombinant construct comprising the first nucleic acid molecule and the second recombinant construct comprising the second nucleic acid molecule), or within a first vector and a second vector. Recombinant constructs and vectors are discussed above. The first and second nucleic acid molecules of the kit are provided as separate molecules, i.e. they are not both located within the same construct or vector.

As noted above, a vector may comprise a selectable marker such that a cell which has taken up the vector can be positively selected. In one embodiment, when the first and second nucleic acid molecules are provided within a first and second vector, the first vector and the second vector each contain a selectable marker. The markers of the first and second vectors are generally different, such that a cell which has taken up both vectors may be selected over a cell which has taken up only one of the two vectors (or neither vector). Suitable selectable markers are discussed above.

The two nucleic acid molecules of the kit (e.g. first and second recombinant constructs or first and second vectors) may be provided in a single container (i.e. in a mixture of the two nucleic acid molecules) or in separate containers. The nucleic acid molecules may be provided in an aqueous solution (e.g. in water or a suitable buffer such as TE buffer) or may be provided in a lyophilised form.

The first chain and the second chain encoded by the nucleic acid molecules in the kit are both derived from either the T1 or the T3 TCR (i.e. the kit does not comprise one nucleic acid molecule encoding a chain derived from the T1 TCR and one nucleic acid molecule encoding a chain derived from the T3 TCR).

Thus in a first embodiment the kit comprises nucleic acid molecules encoding the first and second chains of a T3 TCR-based binding protein, the kit comprising:
(i) a first nucleic acid molecule encoding a first chain comprising a variable domain comprising CDR1, CDR2 and CDR3 which respectively comprise the amino acid sequences set forth in SEQ ID NOs: 16, 17 and 18, in particular wherein the variable domain comprises the amino acid sequence set forth in SEQ ID NO: 22, or an amino acid sequence having at least 90 % or 95 % sequence identity thereto; and
(ii) a second nucleic acid molecule encoding a second chain comprising a variable domain comprising CDR1, CDR2 and CDR3 which respectively comprise the amino acid sequences set forth in SEQ ID NOs: 19, 20 and 21, in particular wherein the variable domain comprises the amino acid sequence set forth in SEQ ID NO: 23, or an amino acid sequence having at least 90 % or 95 % sequence identity thereto.

The first chain may thus be any T3 TCR-based full-length or truncated α-chain, as discussed above, and the second chain may be any T3 TCR-based full-length or truncated β-chain, as discussed above.

In a second embodiment the kit comprises nucleic acid molecules encoding the first and second chains of a T1 TCR-based binding protein, the kit comprising:
(i) a first nucleic acid molecule encoding a first chain comprising a variable domain comprising CDR1, CDR2 and CDR3 which respectively comprise the amino acid sequences set forth in SEQ ID NOs: 2, 3 and 4, in particular wherein the variable domain comprises the amino acid sequence set forth in SEQ ID NO: 8, or an amino acid sequence having at least 90 % or 95 % sequence identity thereto; and
(ii) a second nucleic acid molecule encoding a second chain comprising a variable domain comprising CDR1, CDR2 and CDR3 which respectively comprise the amino acid sequences set forth in SEQ ID NOs: 5, 6 and 7, in particular wherein the variable domain comprises the amino acid sequence set forth in SEQ ID NO: 9, or an amino acid sequence having at least 90 % or 95 % sequence identity thereto.

The first chain may thus be any T1 TCR-based full-length or truncated α-chain, as discussed above, and the second chain may be any T1 TCR-based full-length β-chain, as discussed above.

Sequence identity may be assessed by any convenient method. However, for determining the degree of sequence identity between sequences, computer programmes that make pairwise or multiple alignments of sequences are useful, for instance EMBOSS Needle or EMBOSS stretcher (both Rice, P. et al., Trends Genet., 16, (6) pp276-277, 2000) may be used for pairwise sequence alignments while Clustal Omega (Sievers F et al., Mol. Syst. Biol. 7:539, 2011) or MUSCLE (Edgar, R.C., Nucleic Acids Res. 32(5):1792-1797, 2004) may be used for multiple sequence alignments, though any other appropriate programme may be used. Another suitable alignment programme is BLAST, using the blastp algorithm for protein alignments and the blastn algorithm for nucleic acid alignments. Whether the alignment is pairwise or multiple, it must be performed globally (i.e. across the entirety of the reference sequence) rather than locally.

Sequence alignments and % identity calculations may be determined using for instance standard Clustal Omega parameters: matrix Gonnet, gap opening penalty 6, gap extension penalty 1. Alternatively the standard EMBOSS Needle parameters may be used: matrix BLOSUM62, gap opening penalty 10, gap extension penalty 0.5. Any other suitable parameters may alternatively be used.

Where sequences have less than 100 % identity to the reference sequences recited herein, the sequences may be altered by substitution, deletion or addition of amino acids relative to the reference sequences (or any combination thereof).

The nucleic acid molecules, constructs and vectors provided herein may be introduced into cells, e.g. immune effector cells, by well-known methods. The first and second nucleic acid molecules of the kit provided herein may similarly be co-introduced into a host cell. The nucleic acid molecules, constructs and vectors may be introduced into target eukaryotic (e.g. human) cells by transfection (e.g. by electroporation, microinjection, lipofection or biolistics, or any other method known in the art) or transduction using a viral vector. The nucleic acid molecules, constructs and vectors may be introduced into target prokaryotic (e.g. bacterial) cells by any method known in the art, e.g. transformation, transduction or conjugation.

Further provided herein is thus a cell comprising the recombinant nucleic acid molecule or vector provided herein, or a pair of nucleic acid molecules comprised in the kit provided herein.

The cell may in particular be an effector cell, or more particularly an immune effector cell, or a precursor or progenitor cell therefor, comprising a recombinant nucleic acid molecule or vector as provided herein, or a pair of nucleic acid molecules from the kit, which encode a binding protein as described above comprising a transmembrane domain. The recombinant nucleic acid molecule, vector or pair of nucleic acid molecules may encode a TCR. Alternatively, a TCR-CAR or chimeric TCR may be encoded. When cells, e.g. effector cells, or immune effector cells, express such a binding protein in their cell membrane, they may be able to recognise and target TdT-expressing cells. Thus a cell comprising such a recombinant nucleic acid molecule, vector or pair of nucleic acid molecules, expresses the encoded specific binding protein in its cell membrane. That is to say, the encoded specific binding protein is expressed and localised at the cell membrane, and is functional. In a particular embodiment, the cell, e.g. an effector, or immune effector cell, expresses a functional TCR in its cell membrane.

By "immune effector cell", as referred to herein, is meant any immune cell which is able to perform effector functions when activated (e.g. cytotoxic target cell killing, cytokine release, etc.), and which is able to express a functional TCR. More generally, an "effector cell" is any cell which is able to perform an effector function. This may be any useful effect exerted by the cell, or any useful property of the cell. Effector cells include stem cells. By "functional TCR" is meant a TCR which is able to initiate immune effector functions upon target recognition. An effector cell (which term includes particularly immune effector cells) may be regarded as, or may include, a cell suitable for therapeutic use, e.g. for use in adoptive cell transfer therapy. Accordingly, an effector cell may alternatively be termed a therapeutic cell, or a cell for therapy.

The term "immune effector cell" may include in particular mature or fully differentiated immune effector cells, but as noted above, also included within the scope of the disclosure and uses herein are precursor (or progenitor) cells therefor, including stem cells (for instance haematopoietic stem cells, HSC), or cells derived from HSC or other stem cells, as well as more committed progenitors. A precursor for an immune effector cell may accordingly be a cell obtained or derived from HSCs contained within the CD34+ population of cells derived from a haematopoietic tissue, e.g. from bone marrow, cord blood, or blood e.g. mobilised peripheral blood, which upon administration to a subject differentiate into mature immune effector cells or which can be induced to differentiate into immune effector cells *in vivo* or *in vitro.* A stem cell may also be an induced pluripotent stem cell (iPSC), and thus a precursor for an immune effector cell, may be an iPSC or a cell obtained or derived from iPSC cells.

In a particular embodiment the immune effector cell is a T cell. The T cell can be any type of T cell. For instance, it may be a cytotoxic T cell (a CD8⁺ T cell), a T helper cell (a CD4⁺ T cell), a natural killer T cell (NKT cell) a naïve T cell, a memory T cell or any other type of T-cell. The T cell may be a T helper cell or a cytotoxic T cell. In another particular embodiment, the immune effector cell is a natural killer cell (NK cell).

Thus the immune effector cell may be selected from a cytotoxic T cell, an NK cell and a T helper cell. In one embodiment, the immune effector cell is an NK cell and the binding protein expressed is a TCR. In this case, additional expression of one or more CD3 chains may be required as disclosed in WO 2016/116601 and WO 2018/129199.

A T cell or NK cell comprising a recombinant nucleic acid molecule, vector or pair of nucleic acid molecules as described herein may be obtained by transfecting or transducing a target T cell or NK cell, as described above. The T cells or NK cells to be transfected may be derived from an existing cell line, or may be primary T cells or NK cells, which are isolated from a subject of interest (who may be a patient to be treated or a donor). The T cells or NK cells may also be activated and stimulated to proliferate *in vitro* (such activation and stimulation to proliferate may be referred to as expansion) before and/or after being modified to express the binding molecule. NK cells may be expanded and activated using the methods described in WO 2014/037422.

T cells can be obtained from a number of sources, including peripheral blood mononuclear cells (PBMCs), bone marrow, lymph node tissue, cord blood, thymus tissue, tissue from a site of infection, ascites, pleural effusion, spleen tissue and tumours. T cells may be isolated using any method known in the art. T cells may be obtained from PBMCs, which may be isolated from buffy coats obtained by density gradient centrifugation of whole blood. A specific subpopulation of T cells, such as CD4⁺ or CD8⁺ T cells can be further isolated by positive or negative selection techniques. For example, enrichment of a T-cell population by negative selection can be accomplished with a combination of antibodies directed to surface markers unique to the negatively-selected cells. Such negative selection may be performed by e.g. fluorescence activated cell sorting (FACS).

NK cells may similarly be obtained from the same sources as T-cells using standard methods known in the art, such as those described above. For instance NK cells from a cell line may be used, e.g. NK-92 cells. Primary NK cells may be isolated from blood, e.g. PBMC or umbilical cord blood (UCB), e.g. as described in Becker et al. (Cancer Immunology, Immunotherapy 65: 477-484, 2016) or Spanholtz et al. (PLoS ONE 6(6): e20740, 2011).

The cell, e.g. effector or immune effector cell used herein may be human. Effector cells, e.g. immune effector cells expressing a TCR (or other membrane-bound binding protein provided herein) may be autologous or allogeneic. That is to say, when the cell, e.g. effector or immune effector cell is for therapeutic use, it may be an autologous cell: i.e. derived from the patient to be treated, which ensures histocompatibility and non-immunogenicity, meaning once genetically modified, it will not induce an immune response from the patient. Alternatively, the cell may be a non-autologous cell for therapeutic use (i.e. it is a donor cell obtained from an individual other than the patient), in which case it may be allogeneic.

A non-autologous cell for therapeutic use may be non-immunogenic, such that it does not, when administered to a subject, generate an immune response which affects, interferes with, or prevents the use of the cells in therapy. Non-autologous cells (e.g. immune effector cells or precursors therefor etc.)may be naturally non-immunogenic if they are HLA-matched to the patient. Non-autologous cells may be rendered non-immunogenic by modification to reduce or eliminate their expression of MHC molecules, e.g. by knocking out or knocking down expression of a gene encoding an MHC protein. The cells may be HLA-negative human cells. In an embodiment, disruption of Class-I MHC expression may be performed by knocking out the gene encoding β₂-microglobulin (β₂-m).

The cells (e.g. effector cells, or more particularly immune effector cells) may alternatively be irradiated prior to being administered to a subject. Without wishing to be bound by theory, it is thought that the irradiation of cells results in the cells only being transiently present in a subject, thus reducing the time available for a subject's immune system to mount an immunological response against the cells. Whilst such cells may express a functional MHC molecule at their cell surface, they may also be considered to be non-immunogenic. Radiation may be from any source of α, β or γ radiation, or may be X-ray radiation or ultraviolet light. A radiation dose of 5-10 Gy may be sufficient to abrogate proliferation. Alternatively, the cells may be modified to express a 'suicide gene', which allows the cells to be inducibly killed or prevented from replicating in response to an external stimulus.

The cell provided herein may alternatively be a production host cell and the recombinant nucleic acid molecule, vector or pair of nucleic acid molecules encodes a soluble binding protein as described above (e.g. a soluble TCR). A production host cell is any cell suitable for use in protein production. Appropriate production hosts are known in the art. The production host may be a prokaryote, e.g. an *E. coli* strain optimised for eukaryotic protein production, such as a Rosetta strain. The production host may be a eukaryotic cell, for instance a fungal cell such as a yeast cell, e.g. *Pichia pastoris.* The production host may be a mammalian cell, such as a human cell or a non-human cell. If a non-human mammalian cell is used, the cell may be optimised for human protein expression. Suitable mammalian cells include Cos cells, e.g. COS-7 cells, HEK293 cells and CHO cells, though any suitable cell line or type may be used. CHO (Chinese hamster ovary) cells are commonly used in the art for protein production, and may be used according to the current disclosure. The gene encoding the specific binding molecule may be codon-optimised for expression in the chosen host.

The cell provided herein may alternatively be a cloning host, used during synthesis of the recombinant nucleic acid or vector provided herein, or one of the pair of nucleic acid molecules provided by the kit. A prokaryotic cell may be used as a cloning host for the nucleic acid molecule, construct or vector described above. Suitable prokaryotic cells for use as cloning hosts include, eubacteria, such as Gram-negative or Gram-positive organisms, for example, Enterobacteriaceae such as *Escherichia,* such as *E. coli,* and *Bacilli* such as *B. subtilis.* A cloning host may alternatively be a eukaryotic cell such as a fungal cell, e.g. *Pichia pastoris,* or a yeast cell, or even a higher eukaryotic cell such as a mammalian cell.

Another aspect of the disclosure provides a method of generating a TdT-specific cell, or more particular an effector cell, or an immune effector cell (i.e. an immune effector cell as provided herein) or a precursor therefor, the method comprising introducing a recombinant nucleic acid molecule as provided herein, a vector as provided herein, or a pair of recombinant nucleic acid molecules comprised in the kit provided herein, into the cell (e..g immune effector cell or precursor therefor. The immune effector cell may be any immune effector cell, as described above, in particular a T cell (such as a cytotoxic T cell or a T helper cell) or an NK cell, and the precursor may include any stem cell e.g. iPSC or cells derived therefrom. The nucleic acid molecule(s) or vector may be introduced into the cell by any method known in the art, as also described above, e.g. by transfection or transduction. The cell may be further modified, as described above (e.g. to render it non-immunogenic or to express further proteins of interest, e.g. as detailed above an NK cell may be modified to express both a TCR and the CD3 chains in order to cause expression of a functional TCR-CD3 complex). The effector cell may also be expanded, as described above. Modification and expansion of cells may be performed in any order.

Further provided herein is a pharmaceutical composition comprising a cell as provided herein, particularly an effector cell, and more particularly an immune effector cell or precursor therefor. Also provided is a pharmaceutical composition comprising a soluble binding protein as provided herein (e.g. a TCR-scFv or a soluble TCR) . Pharmaceutical compositions comprising the cells provided herein can be used in cancer treatment, as described further below. Similarly, pharmaceutical compositions comprising soluble binding proteins as provided herein can be used in cancer treatment or diagnosis, as discussed above and further below.

The pharmaceutical composition provided herein comprises the active pharmaceutical agent (i.e. the cell or soluble binding protein) and at least one pharmaceutically-acceptable diluent, carrier or excipient. The composition may be formulated in any convenient manner according to techniques and procedures known in the pharmaceutical art. The term "pharmaceutically-acceptable" as used herein refers to ingredients that are compatible with other ingredients of the compositions as well as physiologically acceptable to the recipient. The nature of the composition and carriers or excipient materials, dosages etc. may be selected in routine manner according to choice and the desired route of administration, purpose of treatment etc.

The pharmaceutical composition may be prepared for administration to a subject by any suitable means. Such administration may be e.g. oral, nasal or parenteral. Oral administration as used herein includes buccal and sublingual administration. Parenteral administration as defined herein includes subcutaneous, intramuscular, intravenous, intraperitoneal and intradermal administration.

Pharmaceutical compositions as provided herein include liquid solutions or syrups, solid compositions such as powders, granules, tablets or capsules, creams, ointments and any other style of composition commonly used in the art. Suitable pharmaceutically acceptable diluents, carriers and excipients for use in such compositions are well known in the art.

For instance, suitable excipients include lactose, maize starch or derivatives thereof, stearic acid or salts thereof, vegetable oils, waxes, fats and polyols. Suitable carriers or diluents include carboxymethylcellulose (CMC), methylcellulose, hydroxypropylmethylcellulose (HPMC), dextrose, trehalose, liposomes, polyvinyl alcohol, pharmaceutical grade starch, mannitol, lactose, magnesium stearate, sodium saccharin, talcum, cellulose, glucose, sucrose (and other sugars), magnesium carbonate, gelatin, oil, alcohol, detergents and emulsifiers such as polysorbates. Stabilising agents, wetting agents, emulsifiers, sweeteners etc. may also be used.

Liquid pharmaceutical compositions, whether they be solutions, suspensions or other like form, may include one or more of the following: sterile diluents such as water for injection, saline solution (which may be physiological), Ringer's solution, isotonic sodium chloride, fixed oils such as synthetic mono- or diglycerides which may serve as a solvent or suspending medium, polyethylene glycols, glycerin, propylene glycol or other solvents; antibacterial agents such as benzyl alcohol or methyl paraben; antioxidants such as ascorbic acid or sodium bisulfite; chelating agents such as EDTA; buffers such as acetates, citrates or phosphates and agents for the adjustment of tonicity such as sodium chloride or dextrose. A parenteral preparation can be enclosed in ampoules, disposable syringes or multiple dose vials made of glass or plastic. An injectable pharmaceutical composition may be sterile.

Provided herein is a cell, particularly an effector cell and more particularly an immune effector cell or precursor therefor, as provided herein for use in therapy. Similarly, provided herein is a pharmaceutical composition comprising such a cell (as described above) for use in therapy. Also provided herein is a soluble binding protein as provided herein (e.g. a TCR-scFv or a soluble TCR) for use in therapy or for use in diagnosis. Similarly, provided herein is a pharmaceutical composition comprising such a soluble binding protein for use in therapy or for use in diagnosis. In the context of the soluble binding proteins provided herein, a soluble binding protein linked or conjugated to a therapeutic agent (as detailed above) may be used in therapy, and a soluble binding protein linked or conjugated to a diagnostic agent (as detailed above) may be used in diagnosis.

By "therapy" as used herein is meant the treatment of any medical condition. Such treatment may be prophylactic (i.e. preventative), curative (or treatment intended to be curative), or palliative (i.e. treatment designed merely to limit, relieve or improve the symptoms of a condition). The therapy is for treatment of a human subject.

Therapy using the cell or composition comprising the cell is adoptive transfer therapy (alternatively known as adoptive cell transfer). Adoptive transfer therapy can be performed using known techniques. The cells may be formulated for therapy by first harvesting them from their culture medium, and then washing and concentrating the cells in a medium and container system suitable for administration (a pharmaceutically-acceptable carrier) in a treatment-effective amount. Suitable infusion medium can be any isotonic medium formulation, typically normal saline, e.g. Normosol R (Abbott) or Plasma-Lyte A (Baxter), but also 5 % dextrose in water or Ringer's lactate can be utilised. The infusion medium can be supplemented with human serum albumin.

The cells provided for therapeutic use herein, which as described above express a membrane-bound specific binding molecule (e.g. a TCR), may be administered either alone, or as a pharmaceutical composition in combination with diluents and/or with other components such as IL-2 or other cytokines or cell populations. Such pharmaceutical compositions are described above.

The cells provided herein for therapeutic use, and pharmaceutical compositions comprising such cells, may be administered to patients by any suitable route. In particular, the cells may be administered intravenously or by intratumoural injection.

Therapy as described herein may comprise administration of a pharmaceutically effective dose of the cells herein. A pharmaceutically effective dose may for example be in the range of 1 x 10⁵ to 1 x 10¹⁰ cells expressing the binding protein (e.g. TCR), or more. A pharmaceutically effective dose may for example be in the range of 1 x 10⁷ to 5 x 10⁹ T cells expressing the TCR. For uses provided herein, the cells are generally in a volume of a litre or less, 500 ml or less, even 250 ml or 100 ml or less. Hence the density of the desired cells is typically greater than 10⁶ cells/ml and generally is greater than 10⁷ cells/ml, generally 10⁸ cells/ml or greater. The clinically relevant number of can be apportioned into multiple infusions that cumulatively equal or exceed 10⁵, 10⁶, 10⁷, 10⁸, 10⁹, 10¹⁰, 10¹¹, or 10¹² cells. For example, 2, 3, 4, 5, 6 or more separate infusions may be administered to a patient, at intervals of 24 or 48 hours, or every 3, 4, 5, 6 or 7 days. Infusions may also be spaced at weekly, fortnightly or monthly intervals, or intervals of 6 weeks or more. The cell compositions may be administered multiple times at dosages within the ranges discussed above. If desired, the treatment may also include administration of mitogens (e.g., PHA) or lymphokines, cytokines, and/or chemokines (e.g., IFN-γ, IL-2, IL-12, TNF-alpha, IL-18, and TNF-beta, GM-CSF, IL-4, IL-13, Flt3-L, RANTES, MΓPTα, etc.) to enhance induction of the immune response.

In therapy using a soluble binding protein as provided herein, dosages etc. may be selected in routine manner according to choice and the desired route of administration, purpose of treatment, etc. Dosages may likewise depend upon the nature of the patient, e.g. age, size and condition, and the type and severity of the patient's disease. Appropriate dosages may be determined by clinical trials. A soluble binding protein as provided herein may alternatively be used in an *in vivo* diagnostic method, in which case the soluble binding protein is linked or conjugated to a diagnostic agent, as discussed above. Appropriate *in vivo* diagnostic methods are known to the skilled physician, and include the utilisation of a soluble binding protein conjugated to a tracer or suchlike, e.g. a radiolabel, in scanning of a patient.

A soluble binding protein may be administered by any route common in the art, e.g. by an oral, nasal or parenteral route as described above.

The present disclosure broadly provides a cell, more particularly an effector cell or an immune effector cell or precursor therefor, expressing a TCR that specifically binds an HLA complex presenting a TdT peptide (or a pharmaceutical composition comprising the aforementioned cell), for use in the treatment of cancer of T cell origin. Similarly, the present disclosure provides a method for treating a cancer of T cell origin, comprising administering a pharmaceutical composition comprising a pharmaceutically effective dose of cells expressing a TCR that specifically binds an HLA complex presenting a TdT peptide.

Further provided herein is a cell, particularly an effector cell and more particularly an immune effector cell or a precursor therefor, as provided herein, a soluble binding protein as provided herein or a pharmaceutical composition as provided herein for use in the treatment of cancer, wherein the cancer expresses TdT. Cancer is defined broadly herein to include any neoplastic condition, whether malignant, pre-malignant or non-malignant. Generally, however, it is a malignant condition. Both solid and non-solid tumours are included and the term "cancer cell" may be taken as synonymous with "tumour cell". A cancer that expresses TdT is a cancer in which the constituent cancer cells express TdT. Whether a cancer expresses TdT can be identified by e.g. analysis of a biopsy sample. A solid or liquid sample may be obtained by standard biopsy procedures, and analysed by histology, e.g. immunohistochemistry using an anti-TdT antibody to identify TdT expression. Other immunological methods may be used to identify TdT expression, e.g. Western blot of a biopsy sample. TdT expression may also be identified by mRNA analysis, e.g. qPCR or RNA-Seq. This aspect of the disclosure can be used to treat cancer in a patient who carries the HLA-A2 gene, meaning that the cancer cells express HLA-A2 (i.e. that the cancer is HLA-A2-positive, or HLA-A*02-positive, optionally HLA-A*02:01-positive). Thus the cancer treated according to the disclosure is HLA-A2-positive.

The cancer may be any cancer, but may in particular be a blood cancer. In a particular embodiment, the blood cancer is acute lymphoblastic leukaemia (ALL). The cancer may be ALL of B cell origin (B-ALL) or ALL of T cell origin (T-ALL). In a particular embodiment, the cancer is T-ALL. In another embodiment, the blood cancer is a non-Hodgkin lymphoma, in particular lymphoblastic lymphoma.

Similarly, provided herein is a method for treating an HLA-A*02 positive cancer that expresses TdT, the method comprising administering a cell, particularly an effector cell and more particularly an immune effector cell or a precursor therefor, soluble binding protein or pharmaceutical composition as provided herein to a subject in need thereof. Such a subject is a human subject suffering from such a cancer (i.e. a cancer that expresses TdT and HLA-A*02. The cancer may in particular be as detailed above.

In general, we also provide a method for treating a TdT-positive cancer in a patient comprising the step of administering a pharmaceutical composition comprising cells, particularly effector cells and more particularly immune effector cells or precursors therefor, expressing a receptor with specific binding to HLA class I-molecules presenting a peptide fragment obtainable from TdT. We also provide a method for treating a TdT-positive cancer in a patient comprising the step of administering a pharmaceutical composition comprising a cytotoxic protein with specific binding to HLA class I-molecules presenting a peptide fragment obtainable from TdT. Suitable peptide fragments obtainable from TdT may comprise 8 to 12 amino acid residues and they may preferably be unique for TdT.

Similarly, provided herein is the use of a cell, particularly an effector cell and more particularly an immune effector cell or precursor therefor, or soluble binding protein as provided herein in the manufacture of a medicament for the treatment of HLA-A*02-positive cancer in a subject, wherein the cancer expresses TdT. The cancer may in particular be a cancer as described above.

Further provided herein is a soluble binding protein as provided herein for use in the diagnosis of HLA-A*02-positive cancer, wherein the cancer expresses TdT. The use of soluble binding proteins in the diagnosis of cancer is detailed above. The cancer may be any cancer, as detailed above with respect to the use of the present disclosure in cancer therapy. Similarly, provided herein is a method of diagnosing cancer, comprising administering a soluble binding protein as provided herein to a subject suspected of having a cancer that is HLA-A*02-positive and expresses TdT.

The present disclosure may be more fully understood from the non-limiting Examples below and in reference to the drawings.

### Examples

### Materials and Methods

### Primary Patient Cells and Cell Lines

Paediatric and young adult relapsed or refractory (r/r) B-ALL patients were enrolled into and treated according to the Chimeric Antigen Receptor (CAR) T cell trials ClinicalTrials.gov Identifier NCT02435849 and NCT03123939; paediatric T-ALL patients according to NOPHO-ALL-2008 (NCT00816049).

Peripheral blood mononuclear cells (PBMCs) were isolated by density-gradient centrifugation (Axis-Shield) from healthy donor buffy coats obtained from the blood bank of Oslo University Hospital. PBMCs were typed for HLA-A2 expression by flow cytometry. Epstein Barr virus-transformed lymphoblastoid cell lines (EBV-LCL) were generated from HLA-A2^{pos} and HLA-A2^{neg} PBMCs as described previously (Tosato et al., Current Protocols in Immunology Chapter 7, Unit 7 22 (2007)). Thymic tissue was cleaned from blood clots, connective tissue, fat tissue, and necrotic tissue, and cut into small pieces that were gently triturated with a 1-ml micropipette attached to a wide-bore pipette tip to release thymocytes into cold RPMI-1640 medium. Thymocytes were washed twice in medium and then cryopreserved.

The following cell lines were obtained from American Type Culture Collection (ATCC) or German Collection of Microorganisms and Cell Cultures (DSMZ): NALM-6, BV173, REH, HPB-ALL, RS4;11, T2, RD, U-2 OS, FM-6, HeLa, HaCaT, MCF7, K562, COLO 688, EA.hy926, EST149, U-87 MG, Daoy, HCT-116, CHP-212 and Phoenix-AMPHO. Most cell lines were purchased from ATCC and DSMZ for the studies and were already authenticated, and cryopreserved aliquots labelled according to passage. Only low passage cell lines were used to start fresh cultures. The identity of cell lines acquired prior to these studies was ascertained by short tandem repeat DNA profiling, a service provided by Labcorp DNA Identification Lab, NC, USA (formerly Genetica, https://celllineauthentication.com/). Cell lines were cultured in humidified cell incubators containing 5 % CO₂ at 37°C in the medium instructed by the supplier, and regularly tested for mycoplasma contamination.

### Induction of Antigen-Specific T Cells

Induction of T cells reactive to TdT peptides and generation of cytotoxic T cell lines and clones was performed as previously described (Ali et al., Nature Protocols 14: 1926-1943, 2019), with some modifications. Briefly, monocytes were isolated from PBMCs of HLA-A2^{pos} healthy donors on day -4 using CD14-reactive microbeads and an AutoMACS Pro Separator (Miltenyi Biotec), and cultured for three days in CellGro GMP DC medium (CellGenix) supplemented with 1 % (vol/vol) human serum (HS, Trina biotech) and 1 % (vol/vol) P/S containing 50 IU/ml Interleukin (IL)-4 (PeproTech) and 800 IU/ml GM-CSF (Genzyme). Subsequently the monocyte-derived-dendritic cells (MoDC) were matured for 14-16 h by adding lipopolysaccharide (LPS; Sigma-Aldrich) and IFN-γ (PeproTech) to final concentrations of 10 ng/ml and 100 IU/ml respectively. On day -1, naive CD8+ T cells were isolated from PBMCs from HLA-A2^{neg} donors by use of an AutoMACS Pro Separator and CD8+ T cell isolation kit mixed with CD45RO- and CD57-reactive beads (Miltenyi Biotec). On day 0, MoDCs were harvested, electroporated with mRNA encoding full-length TdT and co-cultured with naive T cells in DC-T cell medium supplemented with 30 ng/ml IL-21 (PeproTech) at a DC:T cell ratio of 1:4. Parallel control co-cultures were initiated with MoDCs transfected with irrelevant mRNA.

### Sorting and Cloning of pMHC Multimer+ CD8+ T Cells

Single cell cloning of peptide-1 and peptide-3 specific CD8+ T cells was performed as described previously (Ali *et al., supra*). To assess functionality, T cell clones were stimulated with EBV-LCLs pulsed with relevant peptides and the NALM-6 cell line naturally expressing TdT and assessed for up-regulation of CD137.

### TCR Sequencing and Cloning

Paired TCRα and β chains from three clones reactive to peptide-1 and one reactive to peptide-3 were amplified using a protocol described previously that was modified and adapted for the targeted amplification of TCRα and β transcripts (Scheper et al., Nature Medicine 25: 89-94, 2019; Stronen et al., Science 352: 1337-1341, 2016). In brief, RNA was extracted and processed to obtain TCR-specific cDNA. Four pairs of TCRα/β constant domain-specific primers were used to run RT PCR for each clone, followed by addition of poly-G tail and template switch to get double-stranded DNA. Finally, two rounds of nested PCR amplification were performed using additional constant domain primers and adaptor primers annealing to an anchor sequence introduced in the poly-G domain. The Kappa Illumina kit was utilised to prepare libraries, which were later sequenced on an Illumina MiSeq. MiTCR script was used to analyse sequencing data and an in-house Python script TCRprimer was used to reconstruct full-length TCR chains as described previously (Scheper *et al., supra*; Linnemann et al., Nature Medicine 19: 1534-1541, 2013). Output was manually verified for each sample in IMGT/V-Quest (Brochet et al., Nucleic Acids Research 36: W503-508, 2008). Variable TCRα and TCRβ fragments of identified TCRs were codon optimised, synthesised and cloned by Genscript.

### Gene Transfer to Human PBMCs and Cell Lines

T1 and T3 TCRs were transduced into HLA-A2^{pos} donor-derived and patient-derived PBMCs. 1G4 and DMF5 TCRs were also transduced into HLA-A2^{pos} donor-derived PBMCs as controls for *in vivo* experiments. For stimulation of human PBMCs, 6- or 12-well tissue culture-treated plates were coated with anti-CD3 (clone OKT3, eBioscience) and anti-CD28 (clone CD28.6, eBioscience) antibodies. PBMCs (2 x 10⁶ cells/ml) in T cell medium supplemented with IL-7 and IL-15 (5 ng/ml each, PeproTech) were added to antibody-coated plates and incubated at 37°C with 5 % CO₂ for 72 h. For generation of retroviral supernatants, 4 x 10⁶ Phoenix-AMPHO packaging cells were plated in 10 cm petri dishes for 24 h and cells were transfected with γ-retroviral vector DNA, mixed in X-tremeGENE 9 DNA Transfection reagent (Roche Diagnostics) and Opti-MEM. Next day, the medium was refreshed and cells were incubated at 32°C with 5 % CO₂ for 24 h. Subsequently, PBMCs were harvested, re-suspended in T cell medium supplemented with IL-7 and IL-15, mixed with retroviral supernatant, placed in non-tissue culture treated 6-well plates pre-coated with Retronectin (20 µg/ml, Takara) and spinoculated at 900 x g for 60 min. A second spinoculation was performed the following day with fresh retroviral supernatant and transduction efficiency was determined after 3 to 5 days by staining with anti-mouse TCRβ chain antibody and/or pMHC multimer followed by flow cytometry. Prior to functional experiments, cells were cultured for 48-72 h in T cell medium containing low concentrations of cytokines (0.5 ng/ml IL-7 and IL-15). Alternatively, cells were frozen for later experiments.

Retroviral supernatants containing viral DNA encoding full-length HLA-A2 and TdT were also produced as described above and utilised to transduce REH, RD, HeLa, K562, HaCaT, COLO688, EA.hy926 and HPB-ALL cell lines with HLA-A2, and EBV-LCL with TdT. For *in vivo* experiments, the BV173 cell line was stably transduced to express firefly luciferase and green fluorescent protein (GFP), and was designated *BV173ffluc-eGFP.* All transduced cell lines were subsequently purified by FACS sorting, expanded and frozen for use in later experiments.

cDNA for TdT and HLA-A2 were cloned into the pClpA102 vector for mRNA production, as previously described (Kumari et al., PNAS 111: 403-408, 2014).

### Antibodies and Flow Cytometry

Flow cytometry was performed on a BD LSR II flow cytometer (BD Biosciences) and data were analysed using FlowJo (TreeStar) or FACS DIVA (BD Biosciences) software. For surface antibody staining, antibodies were added to cells for 15-20 min on ice followed by washing steps. For intracellular staining, cells were suspended in Cytofix/Cytoperm (BD Bioscience) solution for 20 minutes, washed with Perm/Wash buffer (BD Bioscience) and then stained with antibodies. The following fluorescently conjugated anti-human antibodies were acquired from BD Biosciences or BioLegend unless otherwise specified: Anti-CD14 (HCD14), -HLA-A2 (BB7.2), -CD62L (DREG-56), CD56 (HCD56), -CD57 (HNK-1), -CD45RO (UCHL1), -CD45RA (HI100), -CCR7 (150503), -CD137 (4B4-1), -CD45 (HI30), -TdT (E17-1519), -CD10 (HI10a), -CD19 (HIB19, SJ25C1), -CD38 (HIT2), -CD34 (581), -CD1a (HI149), -CD2 (S5.2), -CD3 (UCHT1, OKT3), -CD8a (RPA-T8), -CD4 (RPA-T4), -CD5 (UCHT2, L17F12), -CD7 (M-T701), anti-mouse CD45 (30-F11) -CD99 (DN16, Bio-Rad) and -CD3 (SK7, eBioscience). Anti-mouse TCRβ chain PE (H57-597, BD Biosciences) was used to test transduction efficiency of T1 and T3 TCRs in human cells and monitor transduced T cells used for *in vivo* treatment in mice. Live/Dead Fixable Near-IR Dead Cell Stain kit (Life Technologies) was used to exclude dead cells in all flow cytometry experiments.

### T Cell Activation Assays

Reactivity of T cell clones and TCR-transduced T cells was investigated by measuring CD137 upregulation or IFN-γ release. Briefly, 100,000 cells/well of indicated target cell lines or primary patient tumour cells were co-incubated with T-cell clones or TCR-transduced PBMC (50,000 cells/well). Where indicated, target cells were pulsed with the specified concentrations of peptide for 1-2 h or electroporated with mRNA encoding full-length TdT, washed, and then co-cultured with effector cells. Following 14-16 h of co-incubation, plates were centrifuged at 400 x g for 3 min. Culture supernatants were harvested for measurement of IFN-γ by ELISA and remaining cells were stained for flow cytometry to measure up-regulation of CD137 on live CD8+ cells. Results are reported either as percentage of CD137+/CD8+ cells (transduction efficiency >90 %) or percentage of CD137+ events among TCR-transduced CD8+ T cells. In some experiments, cells were labelled with 0.75 µM fluorescent cell staining dye, either CellTrace Violet (CTV, Life Technologies) or carboxyfluorescein succinimidyl ester (CSFE, Life Technologies) to distinguish between target and effector cells. The following reagents were acquired from BD Pharmingen or R&D Systems: mouse anti-human IFN-γ capture antibody (NIB42), Biotin Mouse Anti-Human IFN-γ detection antibody (4S.B3), streptavidin-HRP, stabilised tetramethylbenzidine and hydrogen peroxide as substrate solutions, sulfuric acid as stop solution, and recombinant human IFN-γ protein as standard. Assays were performed according to manufacturer's instructions.

### Flow Cytometry-Based Cytotoxicity Assays Using Cell Lines as Targets

For cytotoxicity assays on B- and T-ALL cell lines, 50,000 target cells in T cell medium were co-cultured for 48 h with T1 or T3 TCR-transduced PBMCs in a round bottom 96-well plate in triplicate. Effector cells were defined as TCR-transduced CD8+ T cells in the PBMC product (routinely > 90 % transduction efficiency and 55-65 % CD8+ T cells, the remainder being CD4+ T cells) and effector to target ratio was 1:1. Following co-culture, cells were harvested, washed and stained with human anti-CD3, -CD8, -CD19 and Live/Dead Fixable Near-IR to exclude dead cells. After 15 minutes, cells were washed and re-suspended in 200 µL FACS buffer containing 10,000 CountBright Absolute Counting Beads (Thermo Fisher). Upon data acquisition, an equal number of bead events (5,000) were recorded from every well. Data was normalised and reported as the percentage of the mean live tumour cell number acquired from three parallel wells co-cultured with mock-transduced T cells from the same donor.

### Flow Cytometry-Based Assays for T cell Activation and Cytotoxicity Using Primary Human B-ALL and T-ALL Samples

Peripheral blood or bone marrow samples from B-ALL and T-ALL patients were thawed and resuspended in T cell medium containing low concentrations of IL-7 and IL-15 (0.5 ng/ml). Cells were transferred to round bottom 96-well plates for assays measuring CD137 up-regulation on TCR-transduced T cells or cytotoxicity on target cells. Individualised antibody panels and gating strategies to identify malignant blasts and normal leukocyte populations were designed after reviewing diagnostic phenotyping available in the hospital records. Allogeneic or, for patient 1N, autologous patient-derived T cells transduced with TCRs, were used in the experiments. TCR-transduced cells were pre-labelled with CTV dye to distinguish them from target cells. Wherever indicated, target cells were loaded with relevant peptides for 1-2 h, washed and then co-incubated with TCR-transduced T cells for measurements of CD137 up-regulation, as described above.

For cytotoxicity assays, 50,000 target cells per well were co-incubated with equal numbers of effector cells in 2-4 parallels per condition for 48-72 h and then stained with individualised antibody panels for flow cytometry. CountBright Absolute Counting Beads were utilised for acquisition standardisation, and data was normalised and reported as described above. To visually display flow cytometry plots, we utilised a unsupervised nonlinear dimensionality reduction algorithm such as T-Distributed Stochastic Neighbour Embedding (t-SNE) using FlowJo (TreeStar) software.

### In vivo TdT TCR T cell Activity in two Xenograft B-ALL Cell Line Models

8-10 week old male and female NOD-scid IL2Rgnull (NSG) mice bred in-house were used in these experiments. On day -11, mice were sub-lethally irradiated with 2.5 Gy radiation using a MultiRad225 X-ray irradiator (RPS services). 4 x 10⁶ cells of the human B-ALL cell line BV173 expressing GFP and firefly luciferase were injected on day -10 through the tail vein. After leukaemia was established and confirmed by bioluminescence imaging (BLI) on day -1, mice were treated with TCR-transduced PBMC, transduced with either T1, T3 or a control TCR targeting the cancer testis antigen NY-ESO-1 (1G4) (Rapoport et al., Nature Medicine 21: 914-921, 2015). A separate group of control mice did not receive any T cell injections. Mice were injected daily with 2500 IU IL-2 (R&D Systems) by intra-peritoneal injections, and BLI imaging (IVIS system, PerkinElmer) and blood analysis was performed by flow cytometry at different intervals. For survival analysis, mice were observed for clinical signs of tumour spread, and were sacrificed if they developed more than 20 % weight loss, hunched posture, ruffled fur or limb paralysis. Experiments were terminated two months after T cell injection to avoid the risk of graft-vs-host-disease and surviving mice in treated groups were humanely sacrificed. In one experimental cohort, bone marrow from the surviving T3-treated mice was harvested at the time of sacrifice and processed for flow cytometry to analyse the presence of T cells and tumour cells, as well as expression of TdT and HLA-A2.

### In vivo TdT TCR T cell Activity in a Patient-Derived Xenograft Model

To establish primary human B-ALL xenografted mice, NOD.Cg-Prkdc^{scid} II2rg^{tm1Wjl}/SzJ (NSG; Jackson Laboratory stock 005557) mice 9-15 weeks of age were sub-lethally irradiated by exposure to two doses of 1.65 Gy (X-ray source) 4 hours apart. Viable bone marrow cells from HLA-A2^{pos} B-ALL patient 20O were identified by 7AAD exclusion, and T cell-depleted bone marrow cells were yield sorted on a BD FACS Aria Fusion by exclusion of CD3⁺ cells. 4 x 10⁵ viable CD3 negative bone marrow cells were injected via the tail vein of the NSG mice 4-6 hours after the last irradiation dose. Stable engraftment was confirmed by both PB analysis and bone marrow aspiration from all transplanted mice 18-19 and 20-26 days after transplantation, respectively.

NSG mice were allocated into untreated, DMF5 and T3 T cell groups based on their engraftment levels so that the mean human leukaemic engraftment was comparable among the treatment groups. 7.5 x 10⁶ mTCRβ⁺CD8⁺ T cells transduced with DMF5 TCR or T3 TCR were injected 22-25 days after transplantation of primary B-ALL cells and all groups received daily intraperitoneal (IP) 2500 IU IL-2 (R&D Systems) per mouse. The engraftment was monitored in PB 3 and 10 days after T cell infusion. Upon termination of the mice 11 days after T cell infusion, bone marrow, spleen and PB was subjected to detailed flow cytometry analysis with human anti-CD45, -CD8a, -CD4, -CD3, -CD19, -HLA-A2, -CD10 and mouse anti-CD45, -Ter119 and -TCRβ. For analysis of BM samples, a minimum of 1.8 x 10⁵ events were acquired for all mice and at least 3 x 10⁵ events were acquired for all but two T3 cell-treated mice to determine MRD levels according to NOPHO guidelines.

Cell counts of bone marrow (2 tibias, 2 femurs and 2 crista), and spleen from terminated mice was performed by a Sysmex hematologic cell counter. TrueCount beads (BD Biosciences) were added to whole PB according to manufacturer's instructions and stained for mouse CD45.1 and human CD45 to determine absolute MNC counts per µL blood.

Leukaemia burden and TCR-transduced CD8⁺ cells were quantified for each tissue based on the frequency of human CD45⁺CD19⁺CD10⁺ and human CD45⁺CD3⁺CD8⁺mTCRβ⁺ cells, respectively, in relation to total cell count.

### In vivo Impact of T3 cell Treatment on Normal Human Haematopoiesis in Humanised NSG Mice

NSG mice stably engrafted with HLA-A2^{pos} human cord blood cells were purchased from the Jackson Laboratory. Upon confirmation of human engraftment 21 weeks after transplantation, three mice were terminated. Single cell suspensions from spleens of three engrafted NSG mice were transduced with 1G4 or T3 TCR constructs and expanded as described above. The activity of the NSG-derived 1G4 and T3 cells was validated *in vitro by* performing flow cytometry-based cytotoxicity assays on BV173 cells, in parallel with infusion into the remaining humanised NSG mice. 10⁷ NSG-derived 1G4 or T3 cells were infused via the tail vein into the engrafted mice and the impact of infused cells on normal haematopoiesis was investigated in PB, spleen, thymus and bone marrow 17 days after T cell infusion. As the engrafted mice also contained endogenous cord blood-derived T cells with autocrine IL-2 production potential, supportive infusion of 500 IU daily IP doses of IL-2 was not included for half of the mice (since no differences were observed with or without IL-2 supportive infusions, the data from these groups were pooled). The persistence of infused T cells was monitored in PB, and impact of therapy on mature blood cell lineages was monitored in PB, spleen, thymus and bone marrow by flow cytometry with human anti-CD45, -CD8a, -CD19, -CD33, -CD4, -CD3 and mouse anti-CD45, -Ter119 and TCRβ. The impact on human T cell progenitors in the mouse thymus was investigated through surface and intracellular staining with human anti-TdT, -CD45, -CD8 -CD19, -CD3 (intracellular and surface), -CD4, -HLA-A2 and mouse anti-CD45. Cell counts of bone marrow (2 tibias, 2 femurs and 2 crista), from terminated mice was performed by a Sysmex hematologic cell counter.

### In vitro Impact on Clonogenic Potential of Normal Haematopoietic Progenitors

Bone marrow mononuclear cells were obtained from four HLA-A2^{pos} healthy donors collected at the Karolinska University Hospital with informed consent and ethical approval (EPN 2018/901-31). Viable single CD34⁺ progenitor cells were identified by DAPI and mature lineage exclusion, and sorted on a BD FACS Aria Fusion. 250 CD34⁺Lineage⁻ progenitors were co-cultured with 500 CD4⁻ CD19⁻ (identified by anti-human CD4-PE-Cy5 and anti-human CD19-PE-Cy5 and sorted on BD FACS Aria Fusion) 1G4, T1 or T3 TCR-transduced T cells in StemSpan SFEM (Stem cell technologies) supplemented with 10 % BIT9500 (Stem cell technologies), Penicillin/Streptavidin (100 U/mL; Hyclone Laboratories), 2-mercaptoethanol (2-ME; 0.1 mM; Sigma Aldrich), stem cell factor (SCF; 10 ng/mL), flt3 ligand (FL; 10 ng/mL), thrombopoietin (TPO; 10 ng/mL), interleukin 3 (IL3; 5 ng/mL), granulocyte colony-stimulating factor (G-CSF; 10 ng/mL), granulocyte macrophage colony-stimulating factor (GM-CSF; 10 ng/mL) and erythropoietin (EPO; 1 U/mL) in 37°C, 5 % CO₂. CD34⁺Lineage⁻ progenitors cultured without T cells were used as controls.

After 72 hours the cells from the co-cultures were transferred to cytokine containing methylcellulose (MethoCult H4434, StemCell Technologies) in Iscove's Modified Dulbecco's Medium (IMDM; Gibco) supplemented with 20 % fetal bovine serum (FBS, Sigma Aldrich), L-Glutamine (2 mM; Sigma Aldrich), Penicillin/Streptavidin (100 U/mL) and 2-ME (0.1 mM) to allow for colony generation. After 14 days in methylcellulose, colonies were scored under an inverted microscope as myeloid or erythroid. As a positive control, CD34⁺ Lineage⁻progenitors were externally loaded with 1 µM peptide-1 or peptide-3 in StemSpan SFEM for 2 h followed by 48 h co-culture with or without transduced T cells in the presence of 100 nM peptides, and colonies were scored 10 days after transfer of cells to methylcellulose as described above.

### Statistical Analysis

Statistical analysis was performed using GraphPad Prism version 6 or 7 (GraphPad Software). For BLI signal analysis, ordinary one-way analysis of variance (ANOVA) test with adjustment for multiple comparisons with Tukey's post-test was employed. Survival analysis was performed by Log-rank (Mantel-Cox) test. To determine the difference between the *in vivo* treatment groups in the PDX and humanised NSG mouse models, Kruskal-Wallis ANOVA by Dunn's multiple comparisons test and Mann-Whitney test were performed. P < 0.05 was considered to be statistically significant.

### Results

### TCRs T1 and T3 Specifically Recognise TdT Peptides in an HLA-A2-Restricted Manner

Two TdT-derived peptides identified as HLA-A2 binders (peptide-1 (SEQ ID NO: 1) and peptide-3 (SEQ ID NO: 15)) were used to obtain T cell clones that recognise the TdT protein. The TCR sequences from three analysed peptide-1 reactive clones were identical and named T1, and the sequence from a peptide-3 reactive clone was named T3.

Both TCRs were efficiently expressed in third party peripheral blood (PB) CD8⁺ T cells, as demonstrated by staining with pMHC-multimers or with anti-mouse TCR-β antibodies reactive to the mouse constant region introduced into the TCRs (data not shown). All T3-transduced cells and the majority of T1-transduced cells that stained positively with anti-mouse TCR-β were also pMHC-multimer positive, indicating preferential pairing of introduced TCRα and β-chains after retroviral transduction (data not shown).

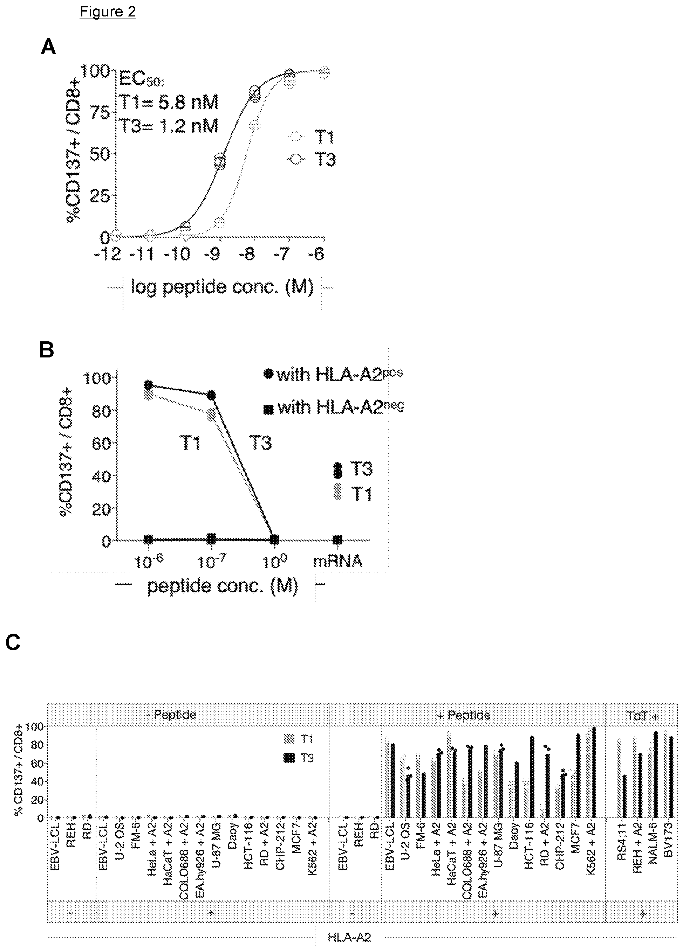

T1 and T3 recognised their cognate peptides with high sensitivity (T1 EC₅₀ = 5.8 nM and T3 EC₅₀ = 1.2 nM, **Fig. 2A****).**

T cells transduced with T1 and T3 TCRs (hereafter called T1 and T3 cells) recognised antigen in an HLA-restricted manner. T1 and T3 cells were activated by HLA-A2^{pos} EBV-LCL presenting externally loaded peptide or endogenously processed antigen derived from mRNA encoding full-length TdT, but not by TdT^{pos} HLA-A2^{neg} EBV-LCLs **(****Fig. 2B****).** Similarly, naturally TdT^{pos} but HLA-A2^{neg} cell lines REH (B-ALL origin) and HPB-ALL (T-ALL origin) activated T1 and T3 cells only when HLA-A*02:01 was introduced. Moreover, we did not observe any TCR activation when we co-cultured T1 and T3 cells with a large panel of human TdT^{neg} cell lines of different tissue origins, unless loaded with relevant peptide and expressing HLA-A2, naturally or by genetic introduction **(****Fig. 2C****).** These results showed that the T1 and T3 TCRs did not react with unintended targets presented on HLA-A2, or on a large variety of other HLA molecules expressed by the cell lines.

### Mapping the Reactivity of TCRs T1 and T3 does not Reveal Cross-Recognised Peptides

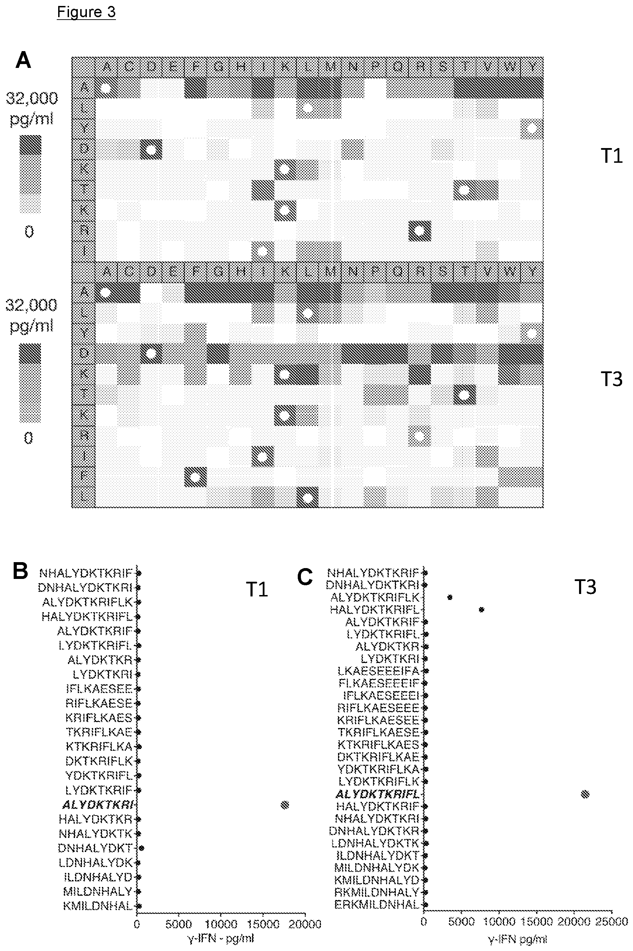

Pre-clinical testing of candidate TCRs should exclude cross-reactivity of potential clinical significance. To this end, we adopted a combined empirical and *in silico* approach to identify potential off-target toxicity. We first synthesised peptide mimotope libraries encoding the sequences of peptide-1 and peptide-3 in which the amino acid residue in each position was replaced by all other natural amino acids, one at a time. T1 and T3 cells were combined with target cells loaded with single mimotopes from the relevant library. Resultant T cell activation, measured by production of IFN-γ or up-regulation of CD137, was highly correlated **(****Fig. 3A****).** Next, we queried the curated human proteome databases UniProtKB/Swiss-Prot and Protein Data Bank for all combinations of aa-substitutions that induced reactivity in T1 or T3 cells, by employing the ScanProsite tool (https://prosite.expasy.org/scanprosite/). The search did not identify any naturally occurring 9-mer or 11-mer peptides in the human proteome that matched those combinations.

When searching the non-curated database UniProtKB/TrEMBL, containing approximately 261 times more entries than UniProtKB/Swiss-Prot, we found one peptide derived from small integral membrane protein 19 (GLFMYAKRIFG) that matched reactivity combinations for T3. However, upon functional challenge, this peptide did not induce any response in T3 cells (not shown). Taken together, the data provided no evidence for off-target reactivities by T1 and T3. Moreover, peptides shifted upstream and/or downstream of the peptide-1 and -3 sequences in the TdT protein sequence failed to activate T1 and T3 cells **(****Fig. 3B-C),** unless longer variants encompassing the whole cognate peptide sequence were used. Peptide-1 moreover failed to activate T3 cells, whereas peptide-3 activated T1 cells only at high concentrations, likely due to breakdown of peptide-3 generating peptide-1 in culture.

### T1 and T3 Mediate Rejection of Disseminated Leukaemia in a Mouse Model

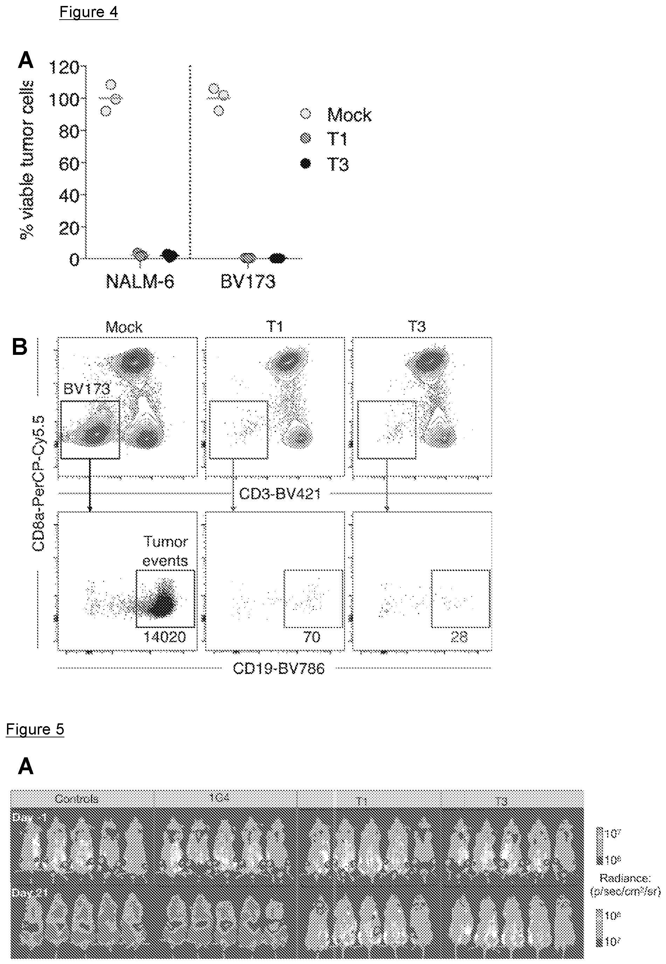

Next, we investigated if T1 and T3 cells could kill leukaemia cell lines naturally expressing TdT. T1 and T3 cells responded strongly to the B-cell leukaemia cell lines BV173 and NALM-6 (naturally TdT^{pos} and HLA-A2^{pos}), as measured by the production of IFN-γ, proliferation and killing (96% - 99% at low effector to target ratios (E:T = 1:1) **Fig. 4A-B).**

To study the efficacy *in vivo,* we engrafted BV173*ffluc-eGFP* cells into NOD-*scid* IL2Rgnull (NSG) mice and started treatment with T1 or T3 cells after leukaemia establishment. A very low (T1) or no (T3) tumour signal was observed in these mice on day 21 (BV173, **Fig. 5A****,** B**)** and day 14 (NALM-6, **Fig. 5D,** E**),** shortly after which untreated and 1G4-treated control mice (control TCR against NY-ESO-1) had to be sacrificed because of high tumour burden **(****Fig. 5C****,** F**).** Notably, none of the mice treated with T3 cells died from leukaemia during the observation-period following injection of leukaemic cells **(****Fig. 5C****,** F**).** Two T3 cell-treated mice in the NALM-6 model died due to unknown reasons, not related to leukaemia spread. Bioluminescence imaging (BLI) remained negative on day 57 in the T3 cell-treated mice in NALM-6 model, consistent with lack of tumour in bone marrow of sacrificed mice (not shown). Only one out of five T3-treated BV173 mice had GFP⁺ tumour cells in the bone marrow upon sacrifice **(****Fig. 5G****).** Control 1G4 TCR-transduced T cells also expanded initially, likely due to injections of IL-2. Survival benefit was also highly significant for BV173 mice treated with T1 cells **(****Fig. 5C****),** but several of these mice eventually succumbed to tumours, consistent with the lower peptide sensitivity for T1 as compared with T3.

### T1 and T3 Eliminate Primary Blasts from B- and T-ALL, but Spare Normal Lymphocytes and Non-Lineage Committed Haematopoietic Progenitor Cells

We next quantified the ability of T1 and T3 cells to selectively recognise human primary ALL cells in samples also containing normal B and T cells and non-lineage committed hematopoietic progenitor cells.. Cryopreserved TdT^{pos} and HLA-A2^{pos} diagnostic samples from 9 B-ALL and 3 T-ALL patients containing variable proportions of leukaemia blasts, normal B and T cells and normal CD34+ haematopoietic progenitor cells (CD34+lin-) were co-cultured with T1 or T3 cells. Following 48-72 hours of co-culture, T3 cells eliminated on average 97 %, and T1 cells 69 %, of leukaemic blasts (T3: mean 97 %, range 92 % - 99.9 %; T1: mean 69 %, range 13 % - 96 %, *n =* 12). In contrast, normal B cells and T cells remained unaffected **(****Fig. 6A-B)** as did non-cancerous CD34⁺lin⁻ cells detected in four patients **(****Fig. 6A****).**

We next showed that introduction of T3 into normal T cells from an HLA-A2^{pos} TdT^{pos} B-ALL patient resulted in T cell activation and elimination of virtually all autologous tumour cells (Fig. 7A). Normal B, T and CD34+lin- progenitor cells were spared (Fig. 7A-B). Taken together, these data indicate a high degree of target selectivity and therapeutic efficacy of T1 and T3 cells against primary leukaemia cells with representative TdT and HLA-A2 expression.

### Further Studies Showing T3 cells Efficiently Eliminate Primary B-ALL cells while Sparing Healthy Haematopoiesis in vivo

We next investigated the efficacy of T3 cell treatment of NSG mice stably engrafted with cells derived from a primary B-ALL leukaemia patient. Untreated mice and mice treated with control MART-1 (DMF5) (Johnson et al., J. Immunol. 177, 6548-6559, 2006) or T3 TCR-transduced T cells showed a high and comparable leukaemia burden in bone marrow at baseline (untreated mean 9.7 ± 3.4 %, DMF5 mean 15.6 ± 5.7 %, T3 mean 15.4 ± 7.2 %), representing a stringent condition for testing of therapeutic efficacy. We utilised T cells matched for HLA-A2 expression but otherwise not HLA-matched with the ALL. Infused T cells contained mainly naïve and central memory T cells and on average 34 % CD4⁺ T cells to resemble a clinical product facilitating anti-tumour reactivity in adoptive T cell therapy. However, a high fraction of naïve T cells also drives alloreactivity, as does the presence of a high burden of leukaemic cells not HLA-matched with the T cells in the mice to be treated. We therefore monitored the mice for the likely development of a graft-versus-leukaemic effect by comparing the leukaemic engraftment in the blood of DMF5 (control TCR)-treated relative to untreated mice, to perform the detailed end-stage analysis before the results could be confounded by significant alloreactivity, which was observed after 10-11 days of treatment. At this experimental end-point leukemia burden in control mouse groups was very high and comparable in the bone marrow (untreated mean 42.9 ± 3.4 %, DMF5 mean 40.1 ± 4.5 %) whereas it was reduced to as little as 0.009 ± 0.005 % following treatment with T3 cells **(****Fig. 8A-D).**

Leukaemia burden in peripheral blood and spleen in untreated and DMF5 cell-treated mice was lower than in bone marrow. Nevertheless, a similar and almost complete elimination of leukaemic cells was observed in mice treated with T3 cells (PB: mean 0.003 ± 0.001 %; spleen: mean 0.002 ± 0.001 %) (data not shown). Taken together, leukaemic burden was reduced to a level considered negative for minimal residual disease (MRD) (<0.1 %) according to criteria set by the NOPHO-2008 protocol (NCT00816049). TCR-transduced T cells were detected in the bone marrow, PB and spleen of all mice (not shown). Mouse haematopoiesis, which is suppressed in the bone marrow of mice with leukaemic engraftment, was significantly higher in T3 than DMF5 mice (not shown).

Normal peripheral B and T cell repertoires are TdT negative and were not affected by T3 or T1 TCRs in *in vitro* killing assays or during expansion of transduced cells (see e.g. **Fig. 6B****).** Studies in which the homeostasis of naïve T cells in normal individuals was measured using retrospective ¹⁴C dating support the view that the naïve T cell repertoire would be sufficiently diverse to sustain peripheral adaptive T cell immunity for life even in young individuals, and that potential toxic effects on thymocytes would therefore not be a major concern even during prolonged therapy. Thymic contribution to the peripheral repertoire is, however, higher in individuals below 20 years of age, raising the possibility that toxicity could be higher in this age group. To address this question, we studied the expression of TdT and HLA-A2 during human thymocyte differentiation. Interestingly, we found that HLA-A2 expression was high only on TdT^{neg} thymocytes, including early double negative (DN) and single positive (SP) cells, whereas HLA-A2 expression was down-regulated concomitantly with up-regulation of TdT on late DN cells transitioning to double positive (DP) cells (data not shown).

We next explored the effect of T3 cell therapy on thymocytes in humanised CD34⁺ NSG mice, which were injected with T3- or 1G4-transduced autologous human T cells harvested from mouse spleen. T3 cells were demonstrated to effectively kill a TdT^{pos} cell line *in vitro* prior to injection (not shown). At the end of the experiment, the fraction of TdT^{pos} thymocytes was similar in T3 and 1G4 treated mice. HLA-A2 levels were similarly low on the TdT^{pos} thymocytes compared to the surface CD3⁺ TdT^{neg} thymocytes in both experimental groups (not shown). Correspondingly, only a minor fraction of human thymocytes, replenished by cord blood progenitors in the mouse thymus, were TdT^{high} *and* HLA-A2^{high} in both groups, as was also observed for thymocytes derived from human thymus (not shown). The experiment was ended on day 17 after T-cell injections when TCR-transduced T cells were still detectable in PB, with the fraction of 1G4-transduced T cells declining faster than T3-transduced T cells in PB, possibly suggesting low-level antigen stimulation of T3 T cells (not shown). No difference between 1G4 and T3 groups was observed upon analysis of the distribution of human myeloid and lymphoid lineages in PB, bone marrow, spleen, and thymus, as well as cellularity in bone marrow (not shown). Further supporting lack of toxicity on haematopoietic stem cells and myeloid progenitor cells, the formation of myeloid and erythroid colonies from adult CD34⁺ bone marrow progenitor cells was not negatively affected following co-culture with T1 or T3 cells, unless co-cultures were performed in the presence of peptide 1 or 3 (not shown).

### Conclusion

Our study shows that T cells transduced with TCRs recognising peptides from the novel cancer target TdT in the context of allogeneic HLA-A2 efficiently killed TdT^{pos}HLA-A2^{pos} patient-derived lymphoblastic leukaemia cells of B- and T-cell origin *in vitro.* The TdT TCRs were highly efficient at killing a large number of TdT^{pos} ALL cell lines as well as primary ALL cells. The TdT TCRs moreover mediated depletion of engrafted leukaemia *in vivo* in three different mouse models of B-ALL. Our data furthermore indicate that the TCRs could be used in therapy without detrimental toxicity.

We have further generated data, not presented here, in which specificity for TdT peptides and HLA-A2-restriction was confirmed by loss of reactivity to TdT^{pos} leukaemia cells in which TdT was knocked out or HLA-A2 was absent, and cognate peptides presented by HLA of primary leukaemia cells were directly identified by mass spectrometry. A variety of HLA-A2^{pos} cell lines that were TdT^{neg} were not recognised, and mapping of TCR-reactivity did not identify any naturally occurring 9- or 11-mer peptide in the human proteome that matched the combinations of substituted peptides to which T1 or T3 were reactive. Taken together, the data indicating high efficacy and specificity of the TdT TCRs combined with the unique expression profile of TdT, open new possibilities for the treatment of TdT^{pos} acute leukaemia, including in patient groups that currently have a dismal prognosis.

**Description of sequences in Sequence Listing**

| **SEQ ID NO** | **Description** |
|---|---|
| 1 | TdT Peptide 1 (T1) |
| 2 | T1 αCDR1 |
| 3 | T1 αCDR2 |
| 4 | T1 αCDR3 |
| 5 | T1 βCDR1 |
| 6 | T1 βCDR2 |
| 7 | T1 βCDR3 |
| 8 | T1 α V domain |
| 9 | T1 β V domain |
| 10 | T1/T3 α extracellular C domain (murine) |
| 11 | T1/T3 β extracellular C domain (murine) |
| 12 | T1 Mature Full-length α chain |
| 13 | T1 Mature Full-length β chain |
| 14 | T1 single chain construct |
| 15 | TdT Peptide 3 (T3) |
| 16 | T3 αCDR1 |
| 17 | T3 αCDR2 |
| 18 | T3 αCDR3 |
| 19 | T3 βCDR1 |
| 20 | T3 βCDR2 |
| 21 | T3 βCDR3 |
| 22 | T3 α V domain |
| 23 | T3 β V domain |
| 24 | T3 Mature Full-length α chain |
| 25 | T3 Mature Full-length β chain |
| 26 | 2A peptide |
| 27 | Human TdT |
| 28 | Human α constant domain |
| 29 | Human β1 constant domain |
| 30 | Human β2 constant domain |
| 31 | Murine α constant domain |
| 32 | Murine β1 constant domain |
| 33 | Murine β2 constant domain |
| 34 | Human α constant domain (Cys-modified) |
| 35 | Human β1 constant domain (Cys-modified) |
| 36 | Human β2 constant domain (Cys-modified) |
| 37 | Murine β1 constant domain (Cys-modified) |
| 38 | Human α TM domain |
| 39 | Human/murine β TM domain |
| 40 | Human CD28 TM domain |
| 41 | Human/murine α cytoplasmic domain |
| 42 | Human β1 cytoplasmic domain |
| 43 | Human β2 cytoplasmic domain |
| 44 | Murine α TM domain |
| 45 | T3 single chain Construct |
| 46 | Murine β1 cytoplasmic domain |
| 47 | Murine β2 cytoplasmic domain |
| 48 | Human CD3ζ signalling domain |
| 49 | Human CD28 co-stimulatory domain |
| 50 | 2A Motif |
| 51 | α-chain leader |
| 52 | β-chain leader T1 |
| 53 | β-chain leader T3 |
| 54 | T1 construct DNA |
| 55 | T3 construct DNA |

## Claims

1. A TCR-derived binding protein capable of specific binding to a human leukocyte antigen (HLA) complex type A2 presenting a peptide having:
(A) the amino acid sequence ALYDKTKRIFL set forth in SEQ ID NO: 15;
wherein the protein comprises an antigen binding unit from a T3 TCR comprising an α-chain variable domain and a β-chain variable domain;
wherein the α-chain variable domain comprises three complementarity determining regions (CDRs): CDR1, CDR2 and CDR3 which respectively comprise the amino acid sequences set forth in SEQ ID NOs: 16, 17 and 18; and
the β-chain variable domain comprises three CDRs: CDR1, CDR2 and CDR3 which respectively comprise the amino acid sequences set forth in SEQ ID NOs: 19, 20 and 21; or
(B) the amino acid sequence ALYDKTKRI set forth in SEQ ID NO: 1,
wherein the protein comprises an antigen binding unit from a T1 TCR comprising an α-chain variable domain and a β-chain variable domain;
wherein the α-chain variable domain comprises three complementarity determining regions (CDRs): CDR1, CDR2 and CDR3 which respectively comprise the amino acid sequences set forth in SEQ ID NOs: 2, 3 and 4; and
the β-chain variable domain comprises three CDRs: CDR1, CDR2 and CDR3 which respectively comprise the amino acid sequences set forth in SEQ ID NOs: 5, 6 and 7.

2. The binding protein of claim 1, wherein:
i) in (A), the α-chain variable domain comprises the amino acid sequence set forth in SEQ ID NO: 22, or an amino acid sequence having at least 90 % sequence identity thereto; and the β-chain variable domain comprises the amino acid sequence set forth in SEQ ID NO: 23, or an amino acid sequence having at least 90 % sequence identity thereto; or
ii) in (B), the α-chain variable domain comprises the amino acid sequence set forth in SEQ ID NO: 8, or an amino acid sequence having at least 90 % sequence identity thereto; and the β-chain variable domain comprises the amino acid sequence set forth in SEQ ID NO: 9, or an amino acid sequence having at least 90 % sequence identity thereto.

3. The binding protein of claims 1 or 2, wherein the binding protein comprises a first chain comprising the α-chain variable domain and a second chain comprising the β-chain variable domain, optionally wherein the first chain further comprises an extracellular α-chain constant domain, and the second chain further comprises an extracellular β-chain constant domain.

4. The binding protein of claim 3, wherein:
i) the extracellular α-chain constant domain comprises the amino acid sequence set forth in SEQ ID NO: 10, or an amino acid sequence having at least 90 % sequence identity thereto;
and the extracellular β-chain constant domain comprises the amino acid sequence set forth in SEQ ID NO: 11, or an amino acid sequence having at least 90 % sequence identity thereto;
ii) the first chain and/or the second chain further comprises a transmembrane domain; and/or
iii) the first chain and/or the second chain further comprises a cytoplasmic domain.

5. The binding protein of claim 3 or 4, wherein:
(a) the first chain is an α-chain comprising the amino acid sequence set forth in SEQ ID NO: 24, and the second chain is a β-chain comprising the amino acid sequence set forth in SEQ ID NO: 25; or
(b) the first chain is an α-chain comprising the amino acid sequence set forth in SEQ ID NO: 12, and the second chain is a β-chain comprising the amino acid sequence set forth in SEQ ID NO: 13.

6. A recombinant nucleic acid molecule encoding a binding protein as defined in any one of claims 1 to 5, optionally wherein the nucleic acid molecule comprises a cDNA molecule.

7. The recombinant nucleic acid molecule of claim 6, wherein the nucleic acid molecule encodes a polypeptide comprising a first chain linked to a second chain, preferably wherein the first chain is linked to the second chain by a self-splicing linker,
optionally wherein the self-splicing linker is a 2A peptide comprising the amino acid sequence set forth in SEQ ID NO: 26, or an amino acid sequence having at least 50 % sequence identity thereto.

8. A vector comprising the recombinant nucleic acid molecule of claim 6 or 7.

9. A kit comprising a first nucleic acid molecule encoding a first chain of a binding protein and a second nucleic acid molecule encoding a second chain of a binding protein, wherein:
(i) the first chain and the second chain respectively comprise α-chain and β-chain variable domains as defined in claim 1(A) or 2(i); or
(ii) the first chain and the second chain respectively comprise α-chain and β-chain variable domains as defined in claim 1(B) or 2(ii).

10. A cell comprising a recombinant nucleic acid molecule as defined in claim 6 or 7, a vector as defined in claim 8, or a pair of recombinant nucleic acid molecules as defined in claim 9, and expressing a binding protein as defined in any one of claims 1 to 5 in its cell membrane.

11. The cell of claim 10, wherein said cell is:
i) an immune effector cell or a precursor therefor; or
ii) a T-cell or a natural killer cell, or a precursor therefor, or wherein said cell is a stem cell, optionally wherein said cell is a T helper cell or a cytotoxic T cell.

12. A pharmaceutical composition comprising a cell as defined in claim 10 or 11.

13. A cell as defined in claim 10 or 11 or a pharmaceutical composition as defined in claim 12 for use in therapy.

14. A cell as defined in claim 10 or 11 or a pharmaceutical composition as defined in claim 12 for use in the treatment of cancer, wherein the cancer expresses terminal deoxynucleotidyl transferase (TdT),
optionally wherein the cancer is acute lymphoblastic leukaemia, preferably of T cell origin or B cell origin.

15. An in-vitro method of generating a terminal deoxynucleotidyl transferase (TdT)-specific cell, the method comprising introducing a recombinant nucleic acid molecule as defined in claim 6 or 7, a vector as defined in claim 8, or a pair of recombinant nucleic acid molecules as defined in claim 9, into the cell,
optionally wherein the cell is a T cell or an NK cell, or a precursor therefor, preferably wherein the T cell is a T helper cell or a cytotoxic T cell.

## Patentansprüche

1. Von TCR-abgeleitetes Bindungsprotein, das zur spezifischen Bindung an einen humanen Leukozytenantigen-(HLA)-Komplex vom Typ A2 fähig ist, der ein Peptid darstellt, das Folgendes aufweist:
(A) die Aminosäuresequenz ALYDKTKRIFL, die in SEQ ID NO: 15 dargelegt ist;
wobei das Protein eine Antigenbindungseinheit von einem T3 TCR umfasst, das eine variable Domäne der α-Kette und eine variable Domäne der β-Kette umfasst;
wobei die variable Domäne der α-Kette drei komplementaritätsbestimmende Regionen (CDRs) umfasst: CDR1, CDR2 und CDR3, die jeweils die Aminosäuresequenzen umfassen, die in SEQ ID NO: 16, 17 und 18 dargelegt sind; und
wobei die variable Domäne der β-Kette drei CDRs umfasst: CDR1, CDR2 und CDR3, die jeweils die Aminosäuresequenzen umfassen, die in SEQ ID NO: 19, 20 und 21 dargelegt sind; oder
(B) die Aminosäuresequenz ALYDKTKRI, die in SEQ ID NO: 1 dargelegt ist,
wobei das Protein eine Antigenbindungseinheit von einem T1 TCR umfasst, die eine variable Domäne der α-Kette und eine variable Domäne der β-Kette umfasst;
wobei die variable Domäne der α-Kette drei komplementaritätsbestimmende Regionen (CDRs) umfasst: CDR1, CDR2 und CDR3, die jeweils die Aminosäuresequenzen umfassen, die in SEQ ID NO: 2, 3 und 4 dargelegt sind; und
wobei die variable Domäne der β-Kette drei CDRs umfasst: CDR1, CDR2 und CDR3, die jeweils die Aminosäuresequenzen umfassen, die in SEQ ID NO: 5, 6 und 7 dargelegt sind.

2. Bindungsprotein nach Anspruch 1, wobei:
i) in (A) die variable Domäne der α-Kette die Aminosäuresequenz umfasst, die in SEQ ID NO: 22 dargelegt ist, oder eine Aminosäuresequenz, die mindestens 90 % Sequenzidentität damit aufweist; und die variable Domäne der β-Kette die Aminosäuresequenz umfasst, die in SEQ ID NO: 23 dargelegt ist, oder eine Aminosäuresequenz, die mindestens 90 % Sequenzidentität damit aufweist; oder
ii) in (B) die variable Domäne der α-Kette die Aminosäuresequenz umfasst, die in SEQ ID NO: 8 dargelegt ist, oder eine Aminosäuresequenz, die mindestens 90 % Sequenzidentität damit aufweist; und die variable Domäne der β-Kette die Aminosäuresequenz umfasst, die in SEQ ID NO: 9 dargelegt ist, oder eine Aminosäuresequenz, die mindestens 90 % Sequenzidentität damit aufweist.

3. Bindungsprotein nach den Ansprüche 1 oder 2, wobei das Bindungsprotein eine erste Kette, die die variable Domäne der α-Kette umfasst, und eine zweite Kette umfasst, die die variable Domäne der β-Kette umfasst, optional, wobei die erste Kette weiter eine extrazelluläre konstante Domäne der α-Kette umfasst und die zweite Kette weiter eine extrazelluläre konstante Domäne der β-Kette umfasst.

4. Bindungsprotein nach Anspruch 3, wobei:
i) die extrazelluläre konstante Domäne der α-Kette die Aminosäuresequenz umfasst, die in SEQ **ID** NO: 10 dargelegt ist, oder eine Aminosäuresequenz, die mindestens 90 % Sequenzidentität damit aufweist;
und die extrazelluläre konstante Domäne der β-Kette die Aminosäuresequenz umfasst, die in SEQ ID NO: 11 dargelegt ist, oder eine Aminosäuresequenz, die mindestens 90 % Sequenzidentität damit aufweist;
ii) die erste Kette und/oder die zweite Kette weiter eine Transmembrandomäne umfassen; und/oder
iii) die erste Kette und/oder die zweite Kette weiter eine zytoplasmatische Domäne umfassen.

5. Bindungsprotein nach Anspruch 3 oder 4, wobei:
(a) die erste Kette eine α-Kette ist, die die Aminosäuresequenz umfasst, die in SEQ ID NO: 24 dargelegt ist, und die zweite Kette eine β-Kette ist, die die Aminosäuresequenz umfasst, die in SEQ ID NO: 25 dargelegt ist; oder
(b) die erste Kette eine α-Kette ist, die die Aminosäuresequenz umfasst, die in SEQ ID NO: 12 dargelegt ist, und die zweite Kette eine β-Kette ist, die die Aminosäuresequenz umfasst, die in SEQ ID NO: 13 dargelegt ist.

6. Rekombinantes Nukleinsäuremolekül, das ein Bindungsprotein kodiert, wie es in einem der Ansprüche 1 bis 5 definiert ist, optional, wobei das Nukleinsäuremolekül ein cDNA-Molekül umfasst.

7. Rekombinantes Nukleinsäuremolekül nach Anspruch 6, wobei das Nukleinsäuremolekül ein Polypeptid kodiert, das eine erste Kette umfasst, die mit einer zweiten Kette verknüpft ist, wobei vorzugsweise die erste Kette mit der zweiten Kette durch einen selbstspleißenden Linker verknüpft ist,
optional, wobei der selbstspleißende Linker ein 2A-Peptid ist, das die Aminosäuresequenz umfasst, die in SEQ ID NO: 26 dargelegt ist, oder eine Aminosäuresequenz, die mindestens 50 % Sequenzidentität damit aufweist.

8. Vektor, umfassend das rekombinante Nukleinsäuremolekül nach Anspruch 6 oder 7.

9. Kit, umfassend ein erstes Nukleinsäuremolekül, das eine erste Kette eines Bindungsproteins kodiert, und ein zweites Nukleinsäuremolekül, das eine zweite Kette eines Bindungsproteins kodiert, wobei:
(i) die erste Kette und die zweite Kette jeweils variable Domänen der α-Kette und β-Kette umfassen, wie sie in Anspruch 1(A) oder 2(i) definiert sind; oder
(ii) die erste Kette und die zweite Kette jeweils variable Domänen der α-Kette und β-Kette umfassen, wie sie in Anspruch 1(B) oder 2(ii) definiert sind.

10. Zelle, umfassend ein rekombinantes Nukleinsäuremolekül, wie es in Anspruch 6 oder 7 definiert ist, einen Vektor, wie er in Anspruch 8 definiert ist, oder ein Paar rekombinanter Nukleinsäuremoleküle, wie sie in Anspruch 9 definiert sind und die in ihrer Zellmembran ein Bindungsprotein exprimieren, wie es in einem der Ansprüche 1 bis 5 definiert ist.

11. Zelle nach Anspruch 10, wobei die Zelle Folgendes ist:
i) eine Immun-Effektorzelle oder ein Vorläufer davon; oder
ii) eine T-Zelle oder eine natürliche Killerzelle oder ein Vorläufer davon, oder wobei die Zelle eine Stammzelle ist, optional, wobei die Zelle eine T-Helferzelle oder eine zytotoxische T-Zelle ist.

12. Pharmazeutische Zusammensetzung, die eine Zelle umfasst, wie sie in Anspruch 10 oder 11 definiert ist.

13. Zelle, wie sie in Anspruch 10 oder 11 definiert ist, oder eine pharmazeutische Zusammensetzung, wie sie in Anspruch 12 definiert ist, zur Verwendung in einer Therapie.

14. Zelle, wie sie in Anspruch 10 oder 11 definiert ist, oder eine pharmazeutische Zusammensetzung, wie sie in Anspruch 12 definiert ist, zur Verwendung in der Behandlung von Krebs, wobei der Krebs terminale Desoxynukleotidyltransferase (TdT) exprimiert,
optional, wobei der Krebs akute Lymphoblastenleukämie ist, vorzugsweise mit T-Zellenursprung oder B-Zellenursprung.

15. In-Vitro-Verfahren zum Erzeugen einer für terminale Desoxynukleotidyltransferase (TdT) spezifischen Zelle, wobei das Verfahren Einführen von einem rekombinanten Nukleinsäuremolekül, wie es in Anspruch 6 oder 7 definiert ist, einem Vektor, wie er in Anspruch 8 definiert ist, oder einem Paar rekombinanter Nukleinsäuremoleküle, wie sie in Anspruch 9 definiert sind, in die Zelle umfasst,
optional, wobei die Zelle eine T-Zelle oder eine NK-Zelle oder ein Vorläufer davon ist, vorzugsweise wobei die T-Zelle eine T-Helferzelle oder eine zytotoxische T-Zelle ist.

## Revendications

1. Protéine de liaison dérivée du TCR capable de se lier spécifiquement à un complexe de type A2 de l'antigène leucocytaire humain (HLA) présentant un peptide ayant :
(A) la séquence d'acides aminés ALYDKTKRIFL indiquée dans SEQ ID NO : 15 ;
dans laquelle la protéine comprend un motif de liaison à l'antigène à partir d'un TCR T3 comprenant un domaine variable de chaîne α et un domaine variable de chaîne β ;
dans laquelle le domaine variable de chaîne α comprend trois régions de détermination de complémentarité (CDR) : CDR1, CDR2 et CDR3 qui comprennent respectivement les séquences d'acides aminés indiquées dans les SEQ ID NO : 16, 17 et 18 ; et
le domaine variable de chaîne β comprend trois CDR : CDR1, CDR2 et CDR3 qui comprennent respectivement les séquences d'acides aminés indiquées dans les SEQ ID NO : 19, 20 et 21 ; ou
(B) la séquence d'acides aminés ALYDKTKRI indiquée dans la SEQ ID NO : 1,
dans laquelle la protéine comprend un motif de liaison à l'antigène à partir d'un TCR T1 comprenant un domaine variable de chaîne α et un domaine variable de chaîne β ;
dans laquelle le domaine variable de chaîne α comprend trois régions de détermination de complémentarité (CDR) : CDR1, CDR2 et CDR3 qui comprennent respectivement les séquences d'acides aminés indiquées dans les SEQ ID NO : 2, 3 et 4 ; et
le domaine variable de chaîne β comprend trois CDR : CDR1, CDR2 et CDR3 qui comprennent respectivement les séquences d'acides aminés indiquées dans les SEQ ID NO : 5, 6 et 7.

2. Protéine de liaison selon la revendication 1, dans laquelle :
i) en (A), le domaine variable de chaîne α comprend la séquence d'acides aminés indiquée dans SEQ ID NO : 22 ou une séquence d'acides aminés présentant au moins 90 % d'identité de séquence avec celle-ci ; et le domaine variable de chaîne β comprend la séquence d'acides aminés indiquée dans SEQ ID NO : 23 ou une séquence d'acides aminés présentant au moins 90 % d'identité de séquence avec celle-ci ; ou
ii) en (B), le domaine variable de chaîne α comprend la séquence d'acides aminés indiquée dans SEQ ID NO : 8 ou une séquence d'acides aminés présentant au moins 90 % d'identité de séquence avec celle-ci ; et le domaine variable de chaîne β comprend la séquence d'acides aminés indiquée dans SEQ ID NO : 9 ou une séquence d'acides aminés présentant au moins 90 % d'identité de séquence avec celle-ci.

3. Protéine de liaison selon les revendications 1 ou la revendication 2, dans laquelle la protéine de liaison comprend une première chaîne comprenant le domaine variable de chaîne α et une seconde chaîne comprenant le domaine variable de chaîne β, dans laquelle facultativement la première chaîne comprend en outre un domaine constant de chaîne α extracellulaire et la seconde chaîne comprend en outre un domaine constant de chaîne β extracellulaire.

4. Protéine de liaison selon la revendication 3, dans laquelle :
i) le domaine constant de chaîne α extracellulaire comprend la séquence d'acides aminés indiquée dans SEQ ID NO : 10 ou une séquence d'acides aminés présentant au moins 90 % d'identité de séquence avec celle-ci ;
et le domaine constant de chaîne β extracellulaire comprend la séquence d'acides aminés présentée dans SEQ ID NO : 11 ou une séquence d'acides aminés présentant au moins 90 % d'identité de séquence avec celle-ci ;
ii) la première chaîne et/ou la seconde chaîne comprennent en outre un domaine transmembranaire ;
iii) la première chaîne et/ou la seconde chaîne comprennent en outre un domaine cytoplasmique.

5. Protéine de liaison selon la revendication 3 ou la revendication 4, dans laquelle :
(a) la première chaîne est une chaîne α comprenant la séquence d'acides aminés indiquée dans SEQ ID NO : 24 et la seconde chaîne est une chaîne β comprenant la séquence d'acides aminés indiquée dans SEQ ID NO : 25 ; ou
(b) la première chaîne est une chaîne α comprenant la séquence d'acides aminés indiquée dans SEQ ID NO : 12 et la seconde chaîne est une chaîne β comprenant la séquence d'acides aminés indiquée dans SEQ ID NO : 13.

6. Molécule d'acide nucléique recombinant codant pour une protéine de liaison selon l'une quelconque des revendications 1 à 5, dans laquelle facultativement la molécule d'acide nucléique comprend une molécule d'ADNc.

7. Molécule d'acide nucléique recombinant selon la revendication 6, dans laquelle la molécule d'acide nucléique code pour un polypeptide comprenant une première chaîne reliée à une seconde chaîne, dans laquelle de préférence la première chaîne est reliée à la seconde chaîne par un lieur à auto-épissage,
dans laquelle facultativement le lieur à auto-épissage est un peptide 2A comprenant la séquence d'acides aminés indiquée dans SEQ ID NO : 26 ou une séquence d'acides aminés présentant au moins 50 % d'identité de séquence avec celle-ci.

8. Vecteur comprenant la molécule d'acide nucléique recombinant selon la revendication 6 ou la revendication 7.

9. Kit comprenant une première molécule d'acide nucléique codant pour une première chaîne d'une protéine de liaison et une seconde molécule d'acide nucléique codant pour une seconde chaîne d'une protéine de liaison, dans lequel :
(i) la première chaîne et la seconde chaîne comprennent respectivement des domaines variables de chaîne α et de chaîne β selon la revendication 1(A) ou la revendication 2(i) ; ou
(ii) la première chaîne et la seconde chaîne comprennent respectivement des domaines variables de chaîne α et de chaîne β selon la revendication 1(B) ou la revendication 2(ii).

10. Cellule comprenant une molécule d'acide nucléique recombinant selon la revendication 6 ou la revendication 7, un vecteur selon la revendication 8, ou une paire de molécules d'acide nucléique recombinant selon la revendication 9, et l'expression d'une protéine de liaison selon l'une quelconque des revendications 1 à 5 dans sa membrane cellulaire.

11. Cellule selon la revendication 10, dans laquelle ladite cellule est :
i) une cellule effectrice immunitaire ou un précurseur de celle-ci ; ou
ii) un lymphocyte T ou une cellule tueuse naturelle ou un précurseur de ceux-ci, ou dans laquelle ladite cellule est une cellule souche, dans laquelle facultativement ladite cellule est un lymphocyte T auxiliaire ou un lymphocyte T cytotoxique.

12. Composition pharmaceutique comprenant une cellule selon la revendication 10 ou la revendication 11.

13. Cellule selon la revendication 10 ou la revendication 11 ou composition pharmaceutique selon la revendication 12 pour une utilisation en thérapie.

14. Cellule selon la revendication 10 ou la revendication 11 ou composition pharmaceutique selon la revendication 12 pour une utilisation dans le traitement du cancer, dans lesquelles le cancer exprime une transférase désoxynucléotidyl terminale (TdT),
dans laquelle facultativement le cancer est une leucémie lymphoblastique aiguë, de préférence d'origine T ou B.

15. Procédé in vitro de génération d'une cellule spécifique à la transférase désoxynucléotidyl terminale (TdT), le procédé comprenant l'introduction d'une molécule d'acide nucléique recombinant selon la revendication 6 ou la revendication 7, un vecteur selon la revendication 8, ou une paire de molécules d'acide nucléique recombinant selon la revendication 9, dans la cellule,
dans lequel facultativement la cellule est un lymphocyte T ou une cellule NK, ou un précurseur de ceux-ci, dans lequel de préférence le lymphocyte T est un lymphocyte T auxiliaire ou un lymphocyte T cytotoxique.
